# EUROPEAN PATENT SPECIFICATION

(11) **EP 3 380 615 B1**
(45) Date of publication and mention of the grant of the patent: **06.01.2021**
(21) Application number: 16805351.0
(22) Date of filing: 25.11.2016
(51) Int. Cl.: C12N 15/113, A61K 31/7088, A61P 29/00

(54) **IL-34 ANTISENSE OLIGONUCLEOTIDES AND METHODS OF USING SAME**
IL-34-ANTISENSE-OLIGONUKLEOTIDE UND VERFAHREN ZUR VERWENDUNG DAVON
OLIGONUCLÉOTIDES ANTISENS IL-34 ET LEURS PROCÉDÉS D'UTILISATION

(30) Priority: 25.11.2015 US 201562260076 P
(43) Date of publication of application: 03.10.2018
(73) Proprietor: Nogra Pharma Limited, Dublin 2 (IE)
(72) Inventor: MCNULTY, Marie, Dublin 2 (IE); VITI, Francesca, 6872 Salorino (CH); BELLINVIA, Salvatore, 6850 Mendrisio (CH)
(74) Representative: Lau, Sarah Jane
(86) International application number: PCT/EP2016/078833
(87) International publication number: WO 2017/089555

(56) References cited:
- WO-A1-2010/149960
- WO-A2-2015/028454
- WO-A2-2015/028454
- WO-A2-2015/028454
- KR-A- 20150 104 275
- KR-A- 20150 104 275
- KR-A- 20150 104 275
- US-B2- 7 618 814
- TAKUYA YOSHINO ET AL: "22 IL-34 Antibody Ameliorates Experimental Colitis by Alternating IL-12p40 Expression in Macrophages", GASTROENTEROLOGY : OFFICIAL PUBLICATION OF THE AMERICAN GASTROENTEROLOGICAL ASSOCIATION, vol. 148, no. 4, April 2015 (2015-04), pages S-8, XP55344242, US ISSN: 0016-5085, DOI: 10.1016/S0016-5085(15)30022-6
- ELEONORA FRANZÈ ET AL: "Interleukin-34 sustains inflammatory pathways in the gut", CLINICAL SCIENCE, vol. 72, no. 3, 24 March 2015 (2015-03-24) , pages 847-280, XP55212604, ISSN: 0143-5221, DOI: 10.1042/CS20150132
- STEPHANIE ZWICKER ET AL: "Interleukin 34: a new modulator of human and experimental inflammatory bowel disease", CLINICAL SCIENCE, vol. 91, no. 3, 21 April 2015 (2015-04-21) , pages 3702-290, XP55212601, ISSN: 0143-5221, DOI: 10.1042/CS20150176
- YAMING WANG ET AL: "Interkeukin-34, a cytokine crucial for the differentiation and maintenance of tissue resident macrophages and Langerhans cells", EUROPEAN JOURNAL OF IMMUNOLOGY, vol. 44, no. 6, 22 May 2014 (2014-05-22), pages 1575-1581, XP55148463, Weinheim ISSN: 0014-2980, DOI: 10.1002/eji.201344365
- TAKUYA YOSHINO ET AL: "Tu1741 Involvement of IL-34 in Intestinal Inflammation of Crohn's Disease", GASTROENTEROLOGY : OFFICIAL PUBLICATION OF THE AMERICAN GASTROENTEROLOGICAL ASSOCIATION, vol. 146, no. 5, 2 May 2014 (2014-05-02), pages S-831, XP55344231, US ISSN: 0016-5085, DOI: 10.1016/S0016-5085(14)63017-1
- YUKO NAKAMICHI ET AL: "IL-34 and CSF-1: similarities and differences", JOURNAL OF BONE AND MINERAL METABOLISM, vol. 31, no. 5, 6 June 2013 (2013-06-06), pages 486-495, XP55344078, JP ISSN: 0914-8779, DOI: 10.1007/s00774-013-0476-3
- JEA-HYUN BAEK ET AL: "IL-34 mediates acute kidney injury and worsens subsequent chronic kidney disease", JOURNAL OF CLINICAL INVESTIGATION, vol. 125, no. 8, 29 June 2015 (2015-06-29) , pages 3198-3214, XP55343929, GB ISSN: 0021-9738, DOI: 10.1172/JCI81166
- Y. NAKAMICHI ET AL: "Spleen serves as a reservoir of osteoclast precursors through vitamin D-induced IL-34 expression in osteopetrotic op/op mice", PROCEEDINGS OF THE NATIONAL ACADEMY OF SCIENCES, vol. 109, no. 25, 19 June 2012 (2012-06-19), pages 10006-10011, XP055344064, US ISSN: 0027-8424, DOI: 10.1073/pnas.1207361109
- TAKUYA YOSHINO ET AL: "IL-34 Antibody Ameliorates Experimental Colitis by Alternating IL-12p40 Expression in Macrophages", GASTROENTEROLOGY, vol. 148, no. 4, April 2015 (2015-04), pages S-8, XP055344242, AMSTERDAM, NL ISSN: 0016-5085, DOI: 10.1016/S0016-5085(15)30022-6
- ELEONORA FRANZÈ ET AL: "Interleukin-34 sustains inflammatory pathways in the gut", CLINICAL SCIENCE, vol. 72, no. 3, 24 March 2015 (2015-03-24) , pages 847-280, XP055212604, ISSN: 0143-5221, DOI: 10.1210/jc.2013-4409
- STEPHANIE ZWICKER ET AL: "Interleukin 34: a new modulator of human and experimental inflammatory bowel disease", CLINICAL SCIENCE, vol. 91, no. 3, 21 April 2015 (2015-04-21) , pages 3702-290, XP055212601, ISSN: 0143-5221, DOI: 10.1073/pnas.0405979102
- YAMING WANG ET AL: "Interkeukin-34, a cytokine crucial for the differentiation and maintenance of tissue resident macrophages and Langerhans cells", EUROPEAN JOURNAL OF IMMUNOLOGY, vol. 44, no. 6, 15 April 2014 (2014-04-15) , pages 1575-1581, XP055148463, ISSN: 0014-2980, DOI: 10.1002/eji.201344365
- EMMA L. MASTELLER ET AL: "Targeting IL-34 in chronic inflammation", DRUG DISCOVERY TODAY, vol. 19, no. 8, 3 June 2014 (2014-06-03), pages 1212-1216, XP055212562, ISSN: 1359-6446, DOI: 10.1016/j.drudis.2014.05.016
- YUKO NAKAMICHI ET AL: "IL-34 and CSF-1: similarities and differences", JOURNAL OF BONE AND MINERAL METABOLISM, vol. 31, no. 5, 6 June 2013 (2013-06-06), pages 486-495, XP055344078, JP ISSN: 0914-8779, DOI: 10.1007/s00774-013-0476-3
- TAKUYA YOSHINO ET AL: "22 IL-34 Antibody Ameliorates Experimental Colitis by Alternating IL-12p40 Expression in Macrophages", GASTROENTEROLOGY : OFFICIAL PUBLICATION OF THE AMERICAN GASTROENTEROLOGICAL ASSOCIATION, vol. 148, no. 4, April 2015 (2015-04), pages S-8, XP55344242, US ISSN: 0016-5085, DOI: 10.1016/S0016-5085(15)30022-6
- ELEONORA FRANZÈ ET AL: "Interleukin-34 sustains inflammatory pathways in the gut", CLINICAL SCIENCE, vol. 72, no. 3, 24 March 2015 (2015-03-24) , pages 847-280, XP55212604, ISSN: 0143-5221, DOI: 10.1042/CS20150132
- STEPHANIE ZWICKER ET AL: "Interleukin 34: a new modulator of human and experimental inflammatory bowel disease", CLINICAL SCIENCE, vol. 91, no. 3, 21 April 2015 (2015-04-21) , pages 3702-290, XP55212601, ISSN: 0143-5221, DOI: 10.1042/CS20150176
- YAMING WANG ET AL: "Interkeukin-34, a cytokine crucial for the differentiation and maintenance of tissue resident macrophages and Langerhans cells", EUROPEAN JOURNAL OF IMMUNOLOGY, vol. 44, no. 6, 22 May 2014 (2014-05-22), pages 1575-1581, XP55148463, Weinheim ISSN: 0014-2980, DOI: 10.1002/eji.201344365
- TAKUYA YOSHINO ET AL: "Tu1741 Involvement of IL-34 in Intestinal Inflammation of Crohn's Disease", GASTROENTEROLOGY : OFFICIAL PUBLICATION OF THE AMERICAN GASTROENTEROLOGICAL ASSOCIATION, vol. 146, no. 5, 2 May 2014 (2014-05-02), pages S-831, XP55344231, US ISSN: 0016-5085, DOI: 10.1016/S0016-5085(14)63017-1
- YUKO NAKAMICHI ET AL: "IL-34 and CSF-1: similarities and differences", JOURNAL OF BONE AND MINERAL METABOLISM, vol. 31, no. 5, 6 June 2013 (2013-06-06), pages 486-495, XP55344078, JP ISSN: 0914-8779, DOI: 10.1007/s00774-013-0476-3
- JEA-HYUN BAEK ET AL: "IL-34 mediates acute kidney injury and worsens subsequent chronic kidney disease", JOURNAL OF CLINICAL INVESTIGATION, vol. 125, no. 8, 29 June 2015 (2015-06-29) , pages 3198-3214, XP55343929, GB ISSN: 0021-9738, DOI: 10.1172/JCI81166
- SILVIA SPECA ET AL: "Cellular and molecular mechanisms of intestinal fibrosis", WORLD JOURNAL OF GASTROENTEROLOGY, vol. 18, no. 28, 28 July 2012 (2012-07-28) , page 3635, XP055181001, ISSN: 1007-9327, DOI: 10.3748/wjg.v18.i28.3635
- Takuya Yoshino ET AL: "22 IL-34 Antibody Ameliorates Experimental Colitis by Alternating IL-12p40 Expression in Macrophages", Gastroenterology, vol. 148, no. 4, 1 April 2015 (2015-04-01) , pages S-8, XP055344242, US ISSN: 0016-5085, DOI: 10.1016/S0016-5085(15)30022-6

## Description

### BACKGROUND

Interleukin-34 (IL-34) is a recently discovered cytokine functionally overlapping macrophage colony stimulating factor (M-CSF, also known as MCSF1 and MCSF-1), a mediator of inflammation and osteoclastogenesis in bone-degenerative diseases such as rheumatoid arthritis. Inflammatory diseases, both acute and chronic, are an important disease category that is still not completely understood.

For example, inflammatory bowel disease is a chronic inflammatory disorder of the gastrointestinal tract suffered by approximately 1.4 million patients in the United States. It is one of the five most prevalent gastrointestinal disease burdens in the United States, with an overall health care cost of more than $1.7 billion. Each year in the United States, inflammatory bowel disease accounts for more than 700,000 physician visits, 100,000 hospitalizations, and disability in 119,000 patients. No medical cure currently exists, so disease management requires a lifetime of care. Antibodies against IL-34 have been used to treat experimental colitis (cf. Takuya Yoshino et al., IL-34 Antibody Ameliorates Experimental Colitis by Alternating IL-12p40 Expression in Macrophages, Gastroenterology 148(4):S-8 (2015)).

The two most common forms of inflammatory bowel disease are Crohn's disease and ulcerative colitis. Although Crohn's disease can affect the entire gastrointestinal tract, it primarily affects the ileum (the distal or lower portion of the small intestine) and the large intestine. Ulcerative colitis primarily affects the colon and the rectum. The etiology of inflammatory bowel disease is not completely understood, although both environmental and genetic factors are believed to play a role in the disease. Environmental components may include alterations in flora of the gut which are affected by exposure to ingested foods and medications.

Inflammatory bowel disease is associated with abdominal pain, vomiting, diarrhea, rectal bleeding, severe cramps, muscle spasms, weight loss, malnutrition, fever, and anemia. Patients with inflammatory bowel disease may also suffer from skin lesions, joint pain, eye inflammation, and liver disorders, and children suffering from ulcerative colitis may suffer from growth defects. Although rarely fatal, these symptoms decrease quality of life for patients.

Thus, there is a pressing need to develop reliable methods of treating inflammatory disorders such as inflammatory bowel disease. There is a further need to identify methods of treatment that provide effective and permanent relief from symptoms across a broad spectrum of patients and which are not associated with negative side effects or cycles of inflammation and remission.

### SUMMARY

Described herein are antisense oligonucleotides against IL-34. The invention provides an antisense oligonucleotide against IL-34 that includes the antisense oligonucleotide sequence of 5'-agctgctcagtgtgaa-3' (SEQ ID NO:5).

In some embodiments, the antisense oligonucleotide may be an antisense oligonucleotide wherein at least one nucleoside linkage of the sequence is a phosphorothioate linkage, a phosphorodithioate linkage, a phosphotriester linkage, an alkylphosphonate linkage, an aminoalkylphosphotriester linkage, an alkylene phosphonate linkage, a phosphinate linkage, a phosphoramidate linkage, and an aminoalkylphosphoramidate linkage, a thiophosphoramidate linkage, thionoalkylphosphonate linkage, a thionoalkylphosphotriester linkage, a thiophosphate linkage, a selenophosphate linkage, or a boranophosphate linkage. In a particular embodiment, at least one internucleoside linkage of the antisense oligonucleotide sequence is a phosphorothioate linkage. In some embodiments, all of the internucleoside linkages of the antisense oligonucleotide sequence are phosphorothioate linkages.

The number of nucleotides included in IL-34 antisense oligonucleotides described herein may vary. In some embodiments, the antisense oligonucleotide is from 22 to 40 nucleotides in length. In some embodiments, the antisense oligonucleotide is from 20 to 30, or 30 to 40 nucleotides in length.

Also described herein are methods of treatment wherein an IL-34 antisense oligonucleotide is administered to a patient in need thereof. For example, described herein are methods of treating an inflammatory disease, comprising administering to a patient in need thereof an effective amount of an IL-34 antisense oligonucleotide described herein. Also described herein is a method of inhibiting inflammatory cytokine production in cells of a patient suffering from an inflammatory disease, comprising administering an effective amount of an IL-34 antisense oligonucleotide described herein.

Also described herein is a method of reducing or inhibiting an IL-34 mediated inflammatory response in cells of a patient suffering from an inflammatory disease, comprising administering an effective amount of an IL-34 antisense oligonucleotide. Also described herein, is a method of treating an inflammatory disease associated with altered IL-34 expression in a patient in need thereof, the method comprising administering an effective amount of an IL-34 antisense oligonucleotide.

Methods described herein may be used to treat inflammatory diseases including, but not limited to, inflammatory bowel disease, rheumatoid arthritis, psoriasis, osteoarthritis, diabetes (Type I and II), tissue or organ rejection, multiple sclerosis, periodontal inflammation, periodontitis, pigmented villonodular synovitis, hepatitis, sinusitis, colon cancer, colorectal cancer, colitis-associated colon cancer, sporadic colorectal cancer, coronary artery disease, Sjogren's syndrome (SS), obesity, chronic inflammation, pulmonary sarcoidosis, skin lesions, a CNS inflammatory disease, or an autoimmune disease. In some embodiments, methods described herein may be used to treat asthma, chronic obstructive pulmonary disease (COPD), or idiopathic pulmonary fibrosis (IPF). In some embodiments, the inflammatory disease is inflammatory bowel disease. In some embodiments, the inflammatory bowel disease is Crohn's disease, gastroduodenal Crohn's disease, Crohn's (granulomatous) colitis, ulcerative colitis, collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, or indeterminate colitis. In particular embodiments, the inflammatory bowel disease is Crohn's disease or ulcerative colitis.

In some embodiments, the IL-34 antisense oligonucleotide is administered topically, parenterally, orally, pulmonarily, intratracheally, intranasally, transdermally, or intraduodenally. In a particular embodiment, the IL-34 antisense oligonucleotide is administered orally.

In some embodiments, the patient in need of treatment is a human.

Also described herein is a pharmaceutically acceptable composition comprising an IL-34 antisense oligonucleotide described herein and a pharmaceutically acceptable carrier. In some embodiments, the pharmaceutical composition is suitable for topical, parenteral, oral, pulmonary, intratracheal, intranasal, transdermal, or intraduodenal administration.

Also described herein is an IL-34 antisense oligonucleotide for use as a medicament.

Also described herein is use of an IL-34 antisense oligonucleotide in the manufacture of a medicament for the treatment of an inflammatory disease. In some embodiments, the inflammatory disease is inflammatory bowel disease, rheumatoid arthritis, psoriasis, osteoarthritis, diabetes (Type I and II), tissue or organ rejection, multiple sclerosis, periodontal inflammation, periodontitis, pigmented villonodular synovitis, hepatitis, sinusitis, colon cancer, colorectal cancer, colitis-associated colon cancer, sporadic colorectal cancer, coronary artery disease, or Sjogren's syndrome (SS), obesity, chronic inflammation, pulmonary sarcoidosis, skin lesions, a CNS inflammatory disease, or an autoimmune disease. Also described herein is use of an IL-34 antisense oligonucleotide in the manufacture of a medicament for the treatment of asthma, chronic obstructive pulmonary disease (COPD), or idiopathic pulmonary fibrosis (IPF). In some embodiments, the inflammatory disease is inflammatory bowel disease. In some embodiments, the inflammatory bowel disease is Crohn's disease, gastroduodenal Crohn's disease, Crohn's (granulomatous) colitis, ulcerative colitis, collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, or indeterminate colitis.

Also described herein is an IL-34 antisense oligonucleotide for use in the treatment of an inflammatory disease. In some embodiments, the inflammatory disease is inflammatory bowel disease, rheumatoid arthritis, psoriasis, osteoarthritis, diabetes (Type I and II), tissue or organ rejection, multiple sclerosis, periodontal inflammation, periodontitis, pigmented villonodular synovitis, hepatitis, sinusitis, colon cancer, colorectal cancer, colitis-associated colon cancer, sporadic colorectal cancer, coronary artery disease, or Sjogren's syndrome (SS), obesity, chronic inflammation, pulmonary sarcoidosis, skin lesions, a CNS inflammatory disease, or an autoimmune disease. Also described herein is an IL-34 antisense oligonucleotide for use in the treatment of asthma, chronic obstructive pulmonary disease (COPD), or idiopathic pulmonary fibrosis (IPF). In some embodiments, the inflammatory disease is inflammatory bowel disease. In some embodiments, the inflammatory bowel disease is Crohn's disease, gastroduodenal Crohn's disease, Crohn's (granulomatous) colitis, ulcerative colitis, collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, or indeterminate colitis.

Also described herein is a method of treating or preventing fibrosis, for example, intestinal fibrosis, pulmonary fibrosis, or liver fibrosis, or preventing collagen deposition, comprising inhibiting IL-34 in a patient suffering from fibrosis. Also described herein is a method of treating or preventing fibrosis or preventing collagen deposition, where the method comprises inhibiting IL-34 in a cell, for example, an intestinal cell. Also described herein is a method of treating or preventing fibrosis or preventing collagen deposition in a patient, where the method comprises administering to the patient an effective amount of a specific inhibitor of IL-34, for example, an antisense oligonucleotide against IL-34.

Also described herein is use of an IL-34 antisense oligonucleotide in the manufacture of a medicament for the treatment fibrosis. In some embodiments, the fibrosis is intestinal fibrosis. In some embodiments, the fibrosis is pulmonary fibrosis. In other embodiments, the fibrosis is renal fibrosis, cardiac fibrosis, endomyocardial fibrosis, myelofibrosis, retroperitoneal fibrosis, or nephrogenic systemic fibrosis.

Also described herein is an IL-34 antisense oligonucleotide for use in the treatment of fibrosis. In some embodiments, the fibrosis is intestinal fibrosis. In some embodiments, the fibrosis is pulmonary fibrosis. In other embodiments, the fibrosis is renal fibrosis, cardiac fibrosis, endomyocardial fibrosis, myelofibrosis, retroperitoneal fibrosis, or nephrogenic systemic fibrosis.

In some embodiments, the patient suffering from fibrosis, for example, intestinal fibrosis, has previously suffered from inflammatory bowel disease or is suffering from inflammatory bowel disease, or inflammatory bowel disease preceded intestinal fibrosis in the patient. In some embodiments, the inflammatory bowel disease is Crohn's disease. In some embodiments, the inflammatory bowel disease is ulcerative colitis.

In some embodiments, the patient suffering from intestinal fibrosis previously suffered from colitis or is suffering from colitis, or colitis preceded intestinal fibrosis in the patient. In some embodiments, the colitis is acute colitis. In some embodiments, the colitis is chronic colitis.

In some embodiments of the invention the patient is a mammal, for example, a primate, for example, a human.

It will be appreciated that the IL-34 antisense oligonucleotide administered to the patient having fibrosis in methods described herein, can be administered by various administration routes. In various embodiments, the IL-34 antisense oligonucleotide can be administered by one or several routes, including orally, topically, parenterally, e.g., by subcutaneous injection, by inhalation spray, or rectally. The term parenteral as used herein includes subcutaneous injections, intrapancreatic administration, and intravenous, intramuscular, intraperitoneal, and intrasternal injection or infusion techniques. In a preferred embodiment, the IL-34 antisense oligonucleotide can be administered orally to the patient having fibrosis, for example, intestinal fibrosis or pulmonary fibrosis,.

Also provided are methods that include administration of a IL-34 antisense oligonucleotide capable of targeting IL-34 RNA for degradation, interfering with RNA splicing, or preventing IL-34 gene expression or protein translation. The IL-34 antisense oligonucleotides of the invention and described herein can target various regions of the human IL-34mRNA for binding. The human IL-34 mRNA sequence is the sequence of NCBI Reference Sequence: NM_001172771.1 (SEQ ID NO: 10), NM_001172772.1 (SEQ ID NO: 11), or NM_152456.2 (SEQ ID NO: 12).

The sequence of the IL-34 antisense oligonucleotide may be selected from multiple sequences capable of targeting IL-34 RNA. In some embodiments of the invention, the antisense oligonucleotide is an antisense oligonucleotide phosphorothioate, *i.e.,* an oligonucleotide where at least some of the internucleotide linkages are phosphorothioate linkages suitable for delivery to cells of a patient. Additionally, antisense oligonucleotides of the invention can include modified nucleotides, for example, nucleotides containing modified bases, for example, 5-methyl-2'-deoxycytidine.

Also described herein is a method of treating fibrosis, for example, intestinal fibrosis, preventing intestinal fibrosis or pulmonary fibrosis, or preventing collagen deposition in a patient includes administering a pharmaceutical composition, for example, a pharmaceutical composition comprising a specific inhibitor of IL-34, for example, an antisense oligonucleotide against IL-34, and a pharmaceutically acceptable carrier. In some embodiments, the pharmaceutical composition is administered parenterally. In some embodiments, the pharmaceutical composition is administered orally. In some embodiments, the pharmaceutical composition includes an enteric coating, for example, an enteric coating comprising an ethylacrylate-methacrylic acid copolymer.

Methods of treating fibrosis (for example, intestinal fibrosis or pulmonary fibrosis), preventing fibrosis (for example, intestinal fibrosis or pulmonary fibrosis), or preventing collagen deposition in a patient include administering varying amounts of an antisense oligonucleotide against IL-34 or a pharmaceutical composition comprising an antisense oligonucleotide against IL-34. Some embodiments include administering at least 1 µg, at least 5 µg, at least 10 µg, at least, 20 µg, at least, 30 µg, at least, 40 µg, at least, 50 µg, at least, 60 µg, at least, 70 µg, at least, 80 µg, at least, 90 or at least 100µg of the antisense oligonucleotide. Some embodiments include administering from 35mg to 500mg, from 1 mg to 10mg, from 10 mg to 20 mg, from 20mg to 30mg, from 30mg to 40mg, from 40mg to 50 mg, from 50mg to 60mg, form 60mg to 70mg, from 70mg to 80mg, from 80mg to 90mg, from 90mg to 100mg, from 100mg to 150mg, from 150mg to 200mg, from 200mg to 250mg, from 250mg to 300mg, from 300mg to 350mg, from 350mg to 400mg, from 400mg to 450mg, from 450mg to 500mg, from 500mg to 600mg, from 600mg to 700mg, from 700mg to 800mg, from 800mg to 900mg, from 900mg to 1g, from 1mg to 50mg, from 20mg to 40mg, or from 1mg to 500mg of the antisense oligonucleotide.

Also provided herein is a method of preventing or treating hepatic fibrosis, pulmonary fibrosis, or intestinal fibrosis, where the method comprises administering to a patient in need thereof a pharmaceutical preparation comprising an IL-34 inhibitor, for example, an IL-34 antisense oligonucleotide such as an IL-34 antisense oligonucleotide disclosed herein. Also provided are methods of preventing or treating renal fibrosis, cardiac fibrosis, endomyocardial fibrosis, idiopathic pulmonary fibrosis, myelofibrosis, retroperitoneal fibrosis, and/or nephrogenic systemic fibrosis, where the method comprises administering to a patient in need thereof a pharmaceutical preparation comprising an IL-34 antisense oligonucleotide such as an IL-34 antisense oligonucleotide disclosed herein.

### BRIEF DESCRIPTION OF THE DRAWINGS

The invention is defined in the claims and any of the following figures that do not fall within the scope of the claims are provided for comparative purposes only.
FIG. 1A shows a bar graph of IL-34 mRNA relative expression in HT-29 cells which were left unstimulated (U), exposed to lipofectamine (L), or transfected with 0.5 µg/mL of an IL-34 antisense oligonucleotide (SEQ ID NO: 1, 2, 3, 4, 5, 6, 7, 8, or 9 as shown in Table 1 of Example 1).
FIG. 1B shows a bar graph of IL-34 mRNA relative expression in HT-29 cells which were left unstimulated (U), exposed to lipofectamine (L), or transfected with 1 µg/mL of an IL-34 antisense oligonucleotide (SEQ ID NO: 1, 2, 3, 4, 5, 6, 7, 8, or 9 as shown in Table 1 of Example 1). FIG. C shows a bar graph of IL-34 mRNA relative expression in HT-29 cells which were left unstimulated (U), exposed to lipofectamine (L), or transfected with 2 µg/mL of an IL-34 antisense oligonucleotide (SEQ ID NO: 1, 2, 3, 4, 5, 6, 7, 8, or 9 as shown in Table 1 of Example 1).
FIG. 2A shows a bar graph of IL-34 mRNA relative expression in normal peripheral blood mononuclear cells (PBMCs) which were left unstimulated (U), Poly (I:C)-stimulated (poly), or Poly (I:C)-stimulated and transfected with 0.5 µg/mL of an IL-34 antisense oligonucleotide (SEQ ID NO: 1, 2, 3, 4, 5, 6, 7, 8, or 9 as shown in Table 1 of Example 1).
FIG. 2B shows a bar graph of IL-34 mRNA relative expression in PBMCs which were left unstimulated (unst), Poly (I:C)-stimulated (poly), or Poly (I:C)-stimulated and transfected with 1 µg/mL of IL-34 antisense oligonucleotide (SEQ ID NO: 1, 2, 3, 4, 5, 6, 7, 8, or 9 as shown in Table 1 of Example 1).
FIG. 2C shows a bar graph of IL-34 mRNA relative expression in PBMCs which were left unstimulated (unst), Poly (I:C)-stimulated (poly), or Poly (I:C)-stimulated and transfected with 2 µg/mL of IL-34 antisense oligonucleotide (SEQ ID NO: 1, 2, 3, 4, 5, 6, 7, 8, or 9 as shown in Table 1 of Example 1).
FIG. 3 is a graph showing normalized IL-34 mRNA levels in paired non-tumoral (NT) and tumoral (T) tissue samples from 12 sporadic CRC patients.
FIG. 4A is a series of micrographs (40x; inset 200x) of non-tumoral (NT) and tumoral (T) colonic tissue samples from sporadic CRC patients probed with either isotype control antibody (IgG, upper panel) or IL-34 antibody (lower panels).
FIG. 4B shows a quantification of the number of IL-34-positive cells in sporadic CRC patient tumoral (T) tissue compared to non-tumoral (NT) tissue per high power field (hpf)(n = 8 patients; NT v. T, p < 0.0001; bars represent standard error of the mean (SEM)).
FIG. 4C shows the results of an ELISA performed on colonic explant tissue taken from the non-tumoral (NT) and tumoral (T) areas of 8 patients with sporadic CRC to measure IL-34 protein levels (NT v. T, p = 0.002; bars represent SEM).
FIG. 4D shows a western blot of tissue extract from non-tumoral (NT) and tumoral (T) areas of 5 patients with sporadic CRC, probed with IL-34 and β-actin antibodies (blot representative of results from 2 experiments).
FIG. 5A is a graph showing the effect of no stimulation (Unst) or recombinant human IL-34 (25 ng/ml, 50 ng/ml, or 100 ng/ml) stimulation on DLD-1 cell proliferation (n = 4; 50 ng/ml v. Unst, p = 0.006; 100 ng/ml v. Unst, p = 0.02; bars represent SEM).
FIG. 5B is a graph showing the effect of recombinant human IL-34 stimulation (50 ng/mL, 48 hours) on DLD-1 cell proliferation following pre-incubation with specific inhibitors of ERK1/2 or p38 (PD98059 or SB202190, respectively) or dimethyl sulfoxide (DMSO) vehicle (n = 4; IL-34 + DMSO v. DMSO, p = 0.01; DMSO v. IL-34 + SB202190, p = 0.01; DMSO + IL-34 v. PD98059 + IL-34, p = 0.006; bars represent SEM).
FIG. 6A is a graph showing percent of DLD-1 cell death in unstimulated (Unst) samples or samples treated with recombinant human IL-34 (25 ng/ml, 50 ng/ml, 100 ng/ml) for 48 hours (n = 4; bars represent mean ± SEM; propidium iodide (PI); Annexin V (AV)).
FIG. 6B is a graph showing the percent of DLD-1 cell death in unstimulated (Unst) samples or samples treated with recombinant human IL-34 and/or Fas Ligand (FasL) as assessed by PI and/or AV staining (n = 4; bars represent mean ± SEM).
FIG 6C is a graph showing the percent of DLD-1 cell death in unstimulated (Unst) samples or samples treated with recombinant human IL-34 and/or FasL as assessed by AV staining alone (n = 4; bars represent mean ± SEM).
FIG. 6D is a graph showing the percent of DLD-1 cell death in unstimulated (Unst) samples or samples treated with recombinant human IL-34 and/or Tumor Necrosis Factor-a (TNF-α) as assessed by PI and/or AV staining (n = 4; bars represent mean ± SEM).
FIG 6E is a graph showing the percent of DLD-1 cell death in unstimulated (Unst) samples or samples treated with recombinant human IL-34 and/or TNF-α as assessed by AV staining alone (n = 4; IL-34 v. IL34 + TNFα, p = 0.03; TNFα v. IL34 + TNFα, p = 0.05; bars represent mean ± SEM).
FIG. 7A is a series of micrographs (200x; inset 400x) of colonic tissue samples from 3 control patients (CTR) and 3 patients with sporadic CRC (CRC) probed with either isotype control antibody (IgG, upper panel) or a macrophage colony-stimulating factor-1 receptor-specific antibody (M-CSFR-1; lower panels).
FIG. 7B is a graph showing normalized M-CSFR-1 mRNA levels in non-tumoral (NT) and tumoral (T) colonic tissue from 6 individuals with sporadic CRC. NT and T data points on the graph from the same patient are connected by lines. Horizontal bars represent the median M-CSFR-1 mRNA values in each of the NT and T groups (NT v. T, p = 0.01).
FIG. 7C is a graph showing normalized receptor-type protein-tyrosine phosphatase zeta (PTP-ζ) mRNA levels in non-tumoral (NT) and tumoral (T) colonic tissue from 6 individuals with sporadic CRC. NT and T data points on the graph from the same patient are connected by lines. Horizontal bars represent the median PTP-ζ mRNA values in each of the NT and T groups (NT v. T, p = 0.03).
FIG. 8A is a graph showing DLD-1 cell proliferation in unstimulated cells (Unst) or following exposure to macrophage colony-stimulating factor -1 (MCSF-1) at 25 ng/ml, 50 ng/ml, or 100 ng/ml (n = 11; bars represent mean ± SEM).
FIG. 8B is a graph showing DLD-1 cell proliferation in unstimulated cells (Unst) or following exposure to human recombinant IL-34 at 25 ng/ml, 50 ng/ml, or 100 ng/ml (n = 7; Unst v. 25 ng/ml, p = 0.001; Unst v. 50 ng/ml, p = 0.001; Unst v. 100 ng/ml, p = 0.001; bars represent mean ± SEM).
FIG. 8C is a graph showing DLD-1 cell death as measured by AV and/or PI staining in unstimulated cells (Unst) or following exposure to MCSF-1 at 25 ng/ml, 50 ng/ml, or 100 ng/ml (n = 6; bars represent mean ± SEM).
FIG. 9 is a graph showing normalized IL-34 mRNA expression levels in DLD-1, HT-29, HCT-116, and NCM-460 cells.
FIG. 10A is an image of a Western blot of DLD-1 cell extracts from cells transfected with a negative control antisense oligonucleotide (NC AS) or an IL-34 antisense oligonucleotide (IL-34 AS) at a concentration of 2 µg/ml for 48 hours, probed with IL-34 or β-actin antibody (representative image from one of four experiments shown).
FIG. 10B is a graph showing percent of cell death in DLD-1 cells transfected with a negative control antisense oligonucleotide (NC AS) or an IL-34 antisense oligonucleotide (IL-34 AS; IL-34 antisense oligonucleotide of SEQ ID NO: 5) at a concentration of 2 µg/ml for 72 hours, stained for AV and PI (n = 4; data is expressed as mean ± SEM).
FIG. 10C is a graph showing relative cell proliferation in DLD-1 cells transfected with a negative control antisense oligonucleotide (NC AS) or an IL-34 antisense oligonucleotide (IL-34 AS; IL-34 antisense oligonucleotide of SEQ ID NO: 5) at a concentration of 2 µg/ml for 72 hours, as evaluated by flow cytometry following staining with CFSE (n = 4; NC AS v. IL-34 AS, p = 0.01; data is expressed as mean ± SEM).
FIG. 10D is an image of a Western blot of DLD-1 cell extracts from cells transfected with either negative control antisense oligonucleotide (NC AS) or an IL-34 antisense oligonucleotide (IL-34 AS; IL-34 antisense oligonucleotide of SEQ ID NO: 5) at a concentration of 2 µg/ml for 72 hours, probed with antibodies against IL-34, total ERK1/2 (ERK1/2), phosphorylated ERK1/2 (pERK1/2), or β-actin (image representative of 4 experiments).
FIG. 11A shows a series of micrographs showing tumoral (T) and non-tumoral (NT) colonic tissue from a colitis-associated colon cancer patient, stained with an M-CSFR-1 antibody (image representative of results from 3 patients; 40x magnification, insets at 200x magnification).
FIG. 11B is a graph showing normalized IL-34 mRNA levels in tumoral (T) and non-tumoral (NT) colonic tissue harvested from wild-type C57BL/6J mice 84 days after azoxymethane (AOM) and dextran sulfate sodium salt (DSS) exposure. NT and T data points from the same animals are connected by lines. Horizontal bars in each column represent median IL-34 mRNA values in the NT and T groups (NT v. T, p = 0.01).
FIG. 12A is a graph showing IL-34 mRNA expression levels in ileal biopsy tissue from control patients (Ileal CTR), patients with inflammatory Crohn's disease (I CD), and patients with fibro-stricturing Crohn's disease (FS CD)(I CD v. Ileal CTR, p = 0.002; FS CD v. Ileal CTR, p = 0.03; n = 5; data are expressed as mean ± SEM).
FIG. 12B shows an image of a Western blot containing protein extracts from 2 control patients (Ileal CTR), 2 patients with I CD, and 2 patients with FS CD probed with antibodies against IL-34 and ERK1/2.
FIG. 12C is a series of micrographs (100X) of paraffin-embedded sections of surgical intestinal samples from 1 normal control (Ileal CTR) and 1 patient suffering from FS CD stained with an IL-34 detection antibody (representative of images from 3 Ileal CTR individuals and 3 FS CD patients).
FIG. 12D is a graph showing normalized IL-34 mRNA expression levels in intestinal fibroblasts from 5 control patients (CTR) and 5 FS CD patients (CTR v. FS CD, p = 0.003; data is represented as the mean ± SEM).
FIG.12E is an image of a Western blot containing protein extract from intestinal fibroblasts of 2 CTR and 2 FS CD patients and probed with antibodies against IL-34 and β-actin.
FIG. 13A is a graph showing normalized M-CSFR-1 mRNA expression levels in ileal biopsy tissue from 5 normal control patients (Ileal CTR), 5 patients suffering from I CD, and 5 patients with FS CD (Ileal CTR v. I CD, p = 0.03; Ileal CTR v. FS CD, p = 0.006; data is expressed as mean ± SEM).
FIG. 13B is a Western blot containing protein extracts of ileal biopsy tissue from 2 control patients (Ileal CTR), 2 patients suffering from I CD, and 2 patients with FS CD, and probed with antibodies against M-CSFR-1 and ERK1/2.
FIG. 14A is a graph showing normalized Collagen Type 1 Alpha 1 chain (Col1A1) levels in fibroblasts that were either left untreated (Unst) or exposed to recombinant human IL-34 protein at a concentration of 50 ng/ml (IL-34) for 6 hours (n = 4; data is expressed as mean ± SEM).
FIG. 14B is a graph showing normalized Collagen Type III Alpha 1 chain (Col3A1) levels in fibroblasts that were either left untreated (Unst) or exposed to recombinant human IL-34 protein at a concentration of 50 ng/ml (IL-34) for 6 hours (Unst v. IL-34, p = 0.03; n = 4; data is expressed as mean ± SEM).
FIG. 14C is a graph showing collagen protein levels in fibroblasts that were either left untreated (Unst) or exposed to recombinant human IL-34 protein at a concentration of 50 ng/ml (IL-34) for 48 hours (Unst v. IL-34, p = 0.01; n = 4; data is expressed as mean ± SEM).

### DETAILED DESCRIPTION

### IL-34 Antisense Oligonucleotides

"Antisense oligonucleotide," as used herein, refers to a short synthetic oligonucleotide sequence complementary to the messenger RNA (mRNA), which encodes for the target protein (*e.g.,* IL-34). Antisense oligonucleotide sequences hybridize to the mRNA producing a double-strand hybrid that can lead to the activation of ubiquitary catalytic enzymes, such as RNase H, which degrades DNA/RNA hybrid strands thus preventing protein translation. Antisense oligonucleotides may include small hairpin RNA's (shRNA's), small interfering RNA's (siRNA's), modified antisense oligonucleotides that include 2'-O-alkyl, peptide nucleic acid (PNA), locked nucleic acid (LNA), or morpholino oligomer chemistries. Antisense oligonucleotides may be single stranded or double-stranded oligonucleotides, where only one strand of the oligonucleotide is complementary to the target sequence.

Antisense oligonucleotides can be designed such that the targeting portion of the incorporated nucleotide sequence of each antisense oligonucleotide is completely or almost completely complementary to the IL-34 mRNA sequence or the mRNA sequence of an IL-34 interaction partner. Incorporation of such complementary or nearly complementary nucleotide sequences allows one to engineer antisense oligonucleotides with a high degree of specificity for a given target. Specificity can be assessed via measurement of parameters such as dissociation constant, or other criteria such as changes in protein or RNA expression levels or other assays that measure IL-34 activity or expression.

An IL-34 antisense oligonucleotide, such as disclosed herein, may be an oligonucleotide sequence of 5 to 100 oligonucleotides in length, for example, 10 to 40 oligonucleotides in length, for example, 14 to 40 oligonucleotides in length, 10 to 30 oligonucleotides in length, for example, 14 to 30 oligonucleotides in length, for example, 14 to 25 or 15 to 22 oligonucleotides in length. An IL-34 antisense oligonucleotide may be an oligonucleotide sequence complementary to a portion of the IL-34 mRNA sequence.

In the present invention, an antisense oligonucleotide against IL-34 comprises a sequence of 5'-AGCTGCTCAGTGTGAA-3' (SEQ ID NO:5).

In an embodiment, at least one or more internucleoside linkage(s) of a disclosed IL-34 antisense oligonucleotide may have a modified linkage, such as a phosphorothioate linkage, a phosphorodithioate linkage, a phosphotriester linkage, an alkylphosphonate linkage, an aminoalkylphosphotriester linkage, an alkylene phosphonate linkage, a phosphinate linkage, a phosphoramidate linkage, and an aminoalkylphosphoramidate linkage, a thiophosphoramidate linkage, thionoalkylphosphonate linkage, a thionoalkylphosphotriester linkage, a thiophosphate linkage, a selenophosphate linkage, and/or a boranophosphate linkage. For example, In some embodiments, one, two or more, *e.g.,* all internucleoside linkages of a disclosed IL-34 antisense oligonucleotide may be phosphorothioate linkages.

In particular embodiments, the antisense oligonucleotide against IL-34 is a phosphorothioate antisense oligonucleotide against IL-34, comprising the sequence of SEQ ID NO:5 (5'-AGCTGCTCAGTGTGAA-3'), where each internucleotide linkage of the antisense oligonucleotide is a phosphorothioate linkage. In some embodiments, the antisense oligonucleotide against IL-34 is a phosphorothioate antisense oligonucleotide against IL-34, comprising the sequence of SEQ ID NO:5 (5'-AGCTGCTCAGTGTGAA-3'), where one or more of the internucleotide linkages of the antisense oligonucleotide are phosphorothioate linkages.

It is contemplated that in an alternative embodiment, a disclosed antisense oligonucleotide targeting IL-34 may optionally have at least one modified nucleobase, e.g., 5-methylcytosine, and/or at least one methylphosphonate nucleotide into the sequence, which is placed, for example, either at only one of the 5' or 3' ends or at both 5' and 3' ends or along the oligonucleotide sequence.

Contemplated IL-34 antisense oligonucleotides may optionally include at least one modified sugar. For example, the sugar moiety of at least one nucleotide constituting the oligonucleotide is a ribose in which the 2'-OH group may be replaced by any one selected from the group consisting of OR, R, R'OR, SH, SR, NH₂, NR₂, N₃, CN, F, Cl, Br, and I (wherein R is an alkyl or aryl and R' is an alkylene).

### Autoimmune and Inflammatory Diseases

The disclosure contemplates , in part, treating inflammatory disorders related to IL-34 activities in a patient in need thereof comprising administering a disclosed antisense compound. Provided herein are methods for treatment of an inflammatory disease in a patient in need thereof, comprising administering a disclosed antisense compound. In some embodiments of the disclosure, an effective amount of a disclosed IL-34 antisense oligonucleotide may be administered to a patient in need thereof to treat an inflammatory disease, for example, to inhibit inflammatory cytokine production in cells of a patient suffering from an inflammatory disease, and/or to reduce or inhibit an IL-34 mediated inflammatory response.

"Inflammatory disease" as used herein, refers to a number of acute and chronic inflammatory disorders including but not limited to inflammatory bowel disease, rheumatoid arthritis, psoriasis, osteoarthritis, diabetes (type I and II), tissue or organ rejection, multiple sclerosis, periodontal inflammation (e.g., periodontitis), pigmented villonodular synovitis, hepatitis, sinusitis, colon cancer, coronary artery disease, or Sjogren's syndrome (SS), obesity, chronic inflammation, pulmonary sarcoidosis, skin lesions, a CNS inflammatory disease, or an autoimmune disease.

Methods of treating skin lesions associated with lupus in patient in need thereof are provided comprising administering a disclosed compound. In some embodiments, treating skin lesions associated with lupus comprises at least one effect selected from reducing the number of skin lesions, reducing the rate of formation of skin lesions, and reducing the severity of skin lesions. Methods of treating lupus and/or lupus nephritis to patient suffering therefrom are provided, that include administering a disclosed antisense compound. Methods of slowing the progression of a kidney condition associated with lupus are provided.

Provided herein are methods of treating, reducing the risk of developing, or delaying the onset of a CNS inflammatory disease in a subject in need thereof are provided comprising administering a disclosed compound. The methods include for example, treating a subject at risk of developing a CNS inflammatory disease; e.g., administering to the subject an effective amount of a disclosed IL-34 antisense. CNS inflammatory disease that can be treated in this manner include multiple sclerosis, experimental autoimmune encephalomyelitis, Alzheimer's disease, amyotrophic lateral sclerosis (ALS) and Parkinson's disease.

Also provided herein are methods of treating, reducing the risk of developing, or delaying the onset of an autoimmune disease in a subject in need thereof comprising administering a disclosed compound. The methods include treating a subject with or at risk of developing an autoimmune disease. Autoimmune diseases that can be treated in this manner include rheumatoid arthritis, type I diabetes, asthma, chronic obstructive pulmonary disease (COPD), and idiopathic pulmonary fibrosis.

### Inflammatory Bowel Disease

The present disclosure also provides methods for treatment of inflammatory bowel disease to a patient in need thereof comprising administering a disclosed compound. "Inflammatory bowel disease," as used herein, refers to a number of chronic inflammatory diseases including Crohn's disease, gastroduodenal Crohn's disease, Crohn's (granulomatous) colitis, ulcerative colitis, collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, and indeterminate colitis. Crohn's disease and ulcerative colitis are the two most common forms of inflammatory bowel disease. Inflammatory bowel disease is an autoimmune disease of the digestive system. Crohn's disease may be localized to any portion of the gastrointestinal tract, including the terminal ileum, and may impact all cell types of the gastrointestinal tract. Ulcerative colitis is localized to the colon and rectum, and affects cells of the mucosa only.

Both environmental and genetic factors are believed to play a role in inflammatory bowel disease, although the identity of such factors is not well-defined. Environmental components may include alterations in flora of the gut which are affected by exposure to ingested foods and medications.

Inflammatory bowel disease is associated with symptoms including abdominal pain, vomiting, diarrhea, rectal bleeding, severe cramps, muscle spasms, weight loss, malnutrition, fever, anemia, skin lesions, joint pain, eye inflammation, liver disorders, arthritis, pyoderma gangrenosum, primary sclerosing cholangitis, and non-thyroidal illness syndrome, and treating these symptoms using a disclosed antisense compound is also contemplated in an embodiment, for example, treating children suffering from ulcerative colitis who may also suffer from growth defects.

### Intestinal Fibrosis

Intestinal fibrosis commonly results from the reaction of intestinal tissue to inflammation, such as chronic inflammation caused by IBD. In IBD, increased levels of SMAD7 block anti-inflammatory gene expression mediated via TGF-β pathway activation and Smad2/3 phosphorylation. Myofibroblasts exposed to increased TGF-β signaling produce increased amounts of collagen. In the context of Crohn's disease, persistent and chronic inflammation promotes fibrotic processes, resulting in the formation of strictures, including small bowel and colonic strictures.

Intestinal fibrosis can be identified by any of a number of imaging techniques, such as contrast-enhanced ultrasonography. See, *e.g.,* Quaia et al. The value of small bowel wall contrast enhancement after sulfur hexafluoride-filled microbubble injection to differentiate inflammatory from fibrotic strictures in patients with Crohn's disease. Ultrasound Med. Biol. 38, 1324-1332 (2012); Nylund et al. Quantitative contrast-enhanced ultrasound comparison between inflammatory and fibrotic lesions in patients with Crohn's disease. Ultrasound Med. Biol. 39, 1197-1206 (2013); Stidham et al. Ultrasound elasticity imaging for detecting intestinal fibrosis and inflammation in rats and humans with Crohn's disease. Gastroenterology 141, 819-826 (2011). MRI techniques such as magnetization transfer MRI can also be used. See, *e.g.,* Maccioni et al. Value of T2-weighted magnetic resonance imaging in the assessment of wall inflammation and fibrosis in Crohn's disease. Abdom. Imaging 37, 944-957 (2012); Adler et al. Magnetization transfer helps detect intestinal fibrosis in an animal model of Crohn disease. Radiology 259, 127-135 (2011); Pazahr et al. Magnetization transfer for the assessment of bowel fibrosis in patients with Crohn's disease: initial experience. Magn. Reson. Mat. Phys. Biol. Med. 26, 291-301 (2013).

### Fibrosis

Methods of preventing or treating fibrosis, such as hepatic fibrosis, pulmonary fibrosis, and/or intestinal fibrosis, form part of this disclosure. Such methods may comprise administering to a patient in need thereof or a patient at risk, a pharmaceutical preparation comprising an IL-34 antisense oligonucleotide such as an IL-34 antisense oligonucleotide disclosed herein. For example, a method of preventing or treating hepatic fibrosis is provided comprising administering to a patient in need thereof an IL-34 antisense oligonucleotide disclosed herein. Alternatively, a method of preventing or treating intestinal fibrosis is provided comprising administering to a patient in need thereof an IL-34 antisense oligonucleotide disclosed herein. Alternatively, a method of preventing or treating pulmonary fibrosis is provided comprising administering to a patient in need thereof an IL-34 antisense oligonucleotide disclosed herein.

Patients treated using an above method may or may not have detectable fibrosis. In some embodiments, the patient has at least about a 5%, 10%, 20%, 30%, 40% or even 50% or more reduction in the amount of fibrosis present in the patient after administering an IL-34 antisense oligonucleotide, after *e.g.* 1 day, 2 days, 1 week, 1 month or 6 months or more. Administering such an IL-34 antisense oligonucleotide may be on, *e.g.,* at least a daily basis. The compound may be administered orally. The delay of clinical manifestation of fibrosis in a patient as a consequence of administering an IL-34 antisense oligonucleotide disclosed here may be at least e.g., 6 months, 1 year, 18 months or even 2 years or more as compared to a patient who is not administered an IL-34 antisense oligonucleotide such as one disclosed herein.

A patient in need may have hepatic fibrosis that has developed into cirrhosis. A patient at risk of hepatic fibrosis may include those patients with hepatitis B, hepatitis C or nonalcoholic steatohepatitis (NASH). NASH is included in the spectrum of nonalcoholic fatty liver diseases, including steatosis and cirrhosis. NASH is a component of the metabolic syndrome, which is characterized by obesity, type 2 diabetes mellitus, and dyslipidemia, and can eventually lead to hepatocellular carcinoma.

Methods of treating disorders associated with hepatic fibrosis are also provided, such as the treatment of at least one of: certain storage diseases and inborn errors of metabolism, such as, alpha 1-antitrypsin deficiency, copper storage diseases (e.g., Wilson's disease), fructosemia, galactosemia, glycogen storage diseases (e.g., Types III, IV, VI, IX and X), iron-overload syndromes (e.g., hemochromatosis), lipid abnormalities (e.g. Gaucher's disease), peroxisomal disorders (e.g., Zellweger syndrome), and tyrosinemia; bacterial infections (e.g., brucellosis); parasitic infections (e.g., echinococcosis); NASH; viral infections (e.g., hepatitis B or hepatitis C, including chronic hepatitis B or C); Budd-Chiari syndrome; heart failure; hepatic veno-occlusive disease; and portal vein thrombosis. Methods of treating congenital hepatic fibrosis are also contemplated. The composition may be administered orally.

Abuse of drugs and/or alcohol has been implicated in cases of hepatic fibrosis. Contemplated herein are methods of treating hepatic fibrosis in a patient with a history of drug and/or alcohol abuse. For example, a patient with a history of abusing at least one of the following: alcohol, amiodarone, chlorpromazine, isoniazid, methotrexate, methyldopa, oxyphenisatin and, tolbutamide.

A patient at risk of intestinal fibrosis may include those patients with ulcerative colitis, inflammatory bowel disease, or Crohn's disease. A patient at risk may also include those patients with an early age at diagnosis of Crohn's or colitis, extensive and/or severe of colonic disease, patients with the presence of primary sclerosing cholangitis, and/or patient's having a family history of cancer.

Methods of treating disorders associated with intestinal fibrosis are also provided, such as the treatment of at least one of: ulcerative colitis, inflammatory bowel disease, or Crohn's disease.

Contemplated herein are methods of preventing or treating renal fibrosis, cardiac fibrosis, endomyocardial fibrosis, idiopathic pulmonary fibrosis, myelofibrosis, retroperitoneal fibrosis, or nephrogenic systemic fibrosis, comprising administering to a patient in need thereof, a pharmaceutical preparation comprising an IL-34 antisense oligonucleotide such as an IL-34 antisense oligonucleotide disclosed herein.

The IL-34 antisense oligonucleotides of the invention can be used alone or in combination with each other where by at least two IL-34 antisense oligonucleotides of the invention are used together in a single composition or as part of a treatment regimen. The IL-34 antisense oligonucleotides of the invention may also be used in combination with other drugs for treating drug and/or alcohol abuse, renal fibrosis, cardiac fibrosis, endomyocardial fibrosis, idiopathic pulmonary fibrosis, myelofibrosis, retroperitoneal fibrosis, or nephrogenic systemic fibrosis, drug and/or alcohol abuse.

### Treatment and Evaluation

A "patient," as described herein, refers to any animal at risk for, suffering from or diagnosed for an inflammatory disease, including, but not limited to, mammals, primates, and humans. In certain embodiments, the patient may be a non-human mammal such as, for example, a cat, a dog, or a horse. A patient may be an individual diagnosed with a high risk of developing an inflammatory disease, someone who has been diagnosed with an inflammatory disease, someone who previously suffered from an inflammatory disease, or an individual evaluated for symptoms or indications of an inflammatory disease, for example, IL-34 expression signal.

"A patient in need," as used herein, refers to a patient suffering from any of the symptoms or manifestations of an inflammatory disease, a patient who may suffer from any of the symptoms or manifestations of an inflammatory disease, or any patient who might benefit from a method of the disclosure for treating an inflammatory disease. A patient in need may include a patient who is diagnosed with a risk of developing an inflammatory disease, a patient who has suffered from an inflammatory disease in the past, or a patient who has previously been treated for an inflammatory disease. Of particular relevance are individuals that suffer from an inflammatory disease associated with increased levels of IL-34 expression or activity.

The terms "treat", "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, *i.e.* preventing the disease from increasing in severity or scope; (c) relieving the disease, *i.e.* causing partial or complete amelioration of the disease; or (d) preventing relapse of the disease, *i.e.* preventing the disease from returning to an active state following previous successful treatment of symptoms of the disease or treatment of the disease.

"Effective amount," as used herein, refers to the amount of an agent that is sufficient to at least partially treat a condition when administered to a patient. The therapeutically effective amount will vary depending on the severity of the condition, the route of administration of the component, and the age, weight, etc. of the patient being treated. Accordingly, an effective amount of a disclosed IL-34 antisense oligonucleotide is the amount of the IL-34 antisense oligonucleotide necessary to treat an inflammatory disease in a patient such that administration of the agent prevents an inflammatory disease from occurring in a subject, prevents an inflammatory disease progression (*e.g*., prevents the onset or increased severity of symptoms of inflammatory bowel disease such as rectal bleeding, anemia, or gastrointestinal inflammation), or relieves or completely ameliorates all associated symptoms of an inflammatory disease, *i.e.* causes regression of the disease.

Efficacy of treatment may be evaluated by means of evaluation of gross symptoms associated with an inflammatory disease, analysis of tissue histology, biochemical assay, imaging methods such as, for example, magnetic resonance imaging, or other known methods. For instance, efficacy of treatment may be evaluated by analyzing gross symptoms of the disease such as changes in abdominal pain, vomiting, diarrhea, rectal bleeding, cramps, muscle spasms, weight loss, malnutrition, fever, anemia or other aspects of gross pathology associated with an inflammatory disease following administration of a disclosed IL-34 antisense oligonucleotide to a patient suffering from an inflammatory disease.

Efficacy of treatment may also be evaluated at the tissue or cellular level, for example, by means of obtaining a tissue biopsy (*e.g.,* a gastrointestinal tissue biopsy) and evaluating gross tissue or cell morphology or staining properties. Biochemical assays that examine protein or RNA expression may also be used to evaluate efficacy of treatment. For instance, one may evaluate IL-34, IL-6, IL-8, TNF-alpha, or levels of another protein or gene product indicative of an inflammatory disease, inflammatory cytokine production, or an IL-34 mediated inflammatory response in dissociated cells or non-dissociated tissue via immunocytochemical, immunohistochemical, Western blotting, or Northern blotting methods, or methods useful for evaluating RNA levels such as quantitative or semi-quantitative polymerase chain reaction. One may also evaluate the presence or level of expression of useful biomarkers found in fecal matter, plasma, or serum to evaluate disease state and efficacy of treatment.

In evaluating efficacy of treatment, suitable controls may be chosen to ensure a valid assessment. For instance, one can compare symptoms evaluated in a patient with an inflammatory disease following administration of a disclosed IL-34 antisense oligonucleotide to those symptoms in the same patient prior to treatment or at an earlier point in the course of treatment or in another patient not diagnosed with the inflammatory disease. Alternatively, one may compare the results of biochemical or histological analysis of gastrointestinal tissue following administration of a disclosed IL-34 antisense oligonucleotide with those of gastrointestinal tissue from the same patient or from an individual not diagnosed with the inflammatory disease or from the same patient prior to administration of the IL-34 antisense oligonucleotide. Additionally, one may compare blood, serum, cell, or fecal samples following administration of the IL-34 antisense oligonucleotide with comparable samples from an individual not diagnosed with the inflammatory disease or from the same patient prior to administration of the IL-34 antisense oligonucleotide.

Validation of IL-34 inhibition may be determined by direct or indirect assessment of IL-34 expression levels or activity. For instance, biochemical assays that measure IL-34 protein or RNA expression may be used to evaluate overall IL-34 inhibition. For instance, one may measure IL-34 protein levels in gastrointestinal tissue by Western blot to evaluate overall IL-34 levels. One may also measure IL-34 mRNA levels by means of Northern blot or quantitative polymerase chain reaction to determine overall IL-34 inhibition. One may also evaluate IL-34 protein levels or levels of another protein indicative of IL-34 signaling activity in dissociated cells, non-dissociated tissue, blood, serum, or fecal matter via immunocytochemical or immunohistochemical methods.

IL-34 inhibition may also be evaluated indirectly by measuring parameters such as macrophage or monocyte generation or proliferation, or measuring alterations in other parameters correlated with changes in IL-34 activity, including MAP kinase phosphorylation and other indicators of signaling activation of the IL-34 receptor - the macrophage colony stimulating factor receptor (M-CSFR-1, also known as MCSFR-1, M-CSFR1, and CSF1R). For instance, one may measure levels of active MAPK1 or MAPK3 phosphorylation in cells of a patient treated with a disclosed IL-34 antisense oligonucleotide as an indication of IL-34 activity in said cells. One may also evaluate the presence or level of expression of useful biomarkers found in plasma, blood, fecal matter or tissue to evaluate efficacy of IL-34 inhibition.

Methods of treatment disclosed herein include methods of inhibiting inflammatory cytokine production. "Inflammatory cytokine production" refers to the expression of cytokines that initiate and/or promote an inflammatory cytokine response. An "inflammatory cytokine response" refers to an immune response that may be characterized by granulocyte recruitment, lymphocyte recruitment, systemic inflammation (especially of the gastrointestinal tract or a portion or portions thereof), fever, tissue destruction, shock, and/or death. An inflammatory cytokine response may be characterized by binding of individual cytokines to their cognate cell surface receptor (*e.g.,* IL-34 binding to CSF1R) and subsequent cascades of intracellular signaling that alter cell functions and gene expression. Inflammatory cytokines include, but are not limited to IL-1, IL-6, IL-8, IL-34, and TNF-alpha. Expression of inflammatory cytokines may occur in, for example, macrophages, monocytes, propia lamina mononuclear cells, or other cells of the gastrointestinal tract or cells of the immune system. Methods of inhibiting inflammatory cytokine production include methods that reduce expression levels of some or all inflammatory cytokines in a patient suffering from an inflammatory disease. Methods of inhibiting inflammatory cytokine production also include methods that reduce expression levels of some or all inflammatory cytokines in cells of a patient suffering from an inflammatory disease.

Methods of the disclosure for inhibiting inflammatory cytokine production include methods of reducing or inhibiting an IL-34 mediated inflammatory response. An "IL-34 mediated inflammatory response," as used herein, refers to an inflammatory response initiated, facilitated, or promoted by IL-34 expression or IL-34 signaling activity. An IL-34 mediated inflammatory response may result in expression of inflammatory cytokines including, but not limited to, IL-34, IL-6, IL-8, or TNF-alpha, and activation of inflammatory cytokine signaling. Additionally, an IL-34 mediated inflammatory response may be characterized by granulocyte recruitment, lymphocyte recruitment, systemic inflammation (especially of the gastrointestinal tract or a portion or portions thereof), fever, tissue destruction, shock, and/or death. An IL-34 mediated inflammatory response may also be characterized by activation of IL-34 signaling, for instance, binding of IL-34 to CSF1R and phosphorylation of downstream MAP kinases. Reducing or inhibiting an IL-34 mediated inflammatory response refers to alleviating any or all of the cellular and systemic changes associated with an IL-34 mediated inflammatory response. For example, a reduction in inflammatory cytokine production, immune cell recruitment, or tissue inflammation would indicate reducing or inhibiting of an IL-34 mediated inflammatory response.

The disclosure also provides methods of inhibiting IL-34 in cells of a patient suffering from an inflammatory disease. IL-34 may be inhibited in any cell in which IL-34 expression or activity occurs, including cells of the gastrointestinal tract, immune system, and blood. Cells of the gastrointestinal tract (including cells of the stomach, duodenum, jejunum, ileum, colon, rectum and anal canal), include columnar epithelial cells, mucosal epithelial cells, zymogenic cells, neck mucus cells, parietal cells, gastrin cells, goblet cells, paneth cells, oligomucus cells, and villus absorptive cells. Cells of the immune system include leukocytes, phagocytes (*e.g*., macrophages, neutrophils, and dendritic cells), monocytes, mast cells, eosinophils, basophils, natural killer cells, innate cells, lymphocytes, B cells, and T cells. Blood cells include red blood cells (erythrocytes) and white blood cells (leukocytes, monocytes, and platelets).

### Pharmaceutical Compositions and Routes of Administration

The present disclosure also provides methods for treating an inflammatory disease via administration of a pharmaceutical composition comprising a disclosed IL-34 antisense oligonucleotide. In another aspect, the disclosure provides a pharmaceutical composition for use in treating an inflammatory disease. The pharmaceutical composition may be comprised of a disclosed antisense oligonucleotide that targets IL-34 and a pharmaceutically acceptable carrier. As used herein the term "pharmaceutical composition" means, for example, a mixture containing a specified amount of a therapeutic compound, *e.g.,* a therapeutically effective amount, of a therapeutic compound in a pharmaceutically acceptable carrier to be administered to a mammal, *e.g*., a human, in order to treat an inflammatory disease. In some embodiments, contemplated herein are pharmaceutical compositions comprising a disclosed IL-34 antisense oligonucleotide and a pharmaceutically acceptable carrier. In another aspect, the disclosure provides use of a disclosed IL-34 antisense oligonucleotide in the manufacture of a medicament for treating an inflammatory disease. "Medicament," as used herein, has essentially the same meaning as the term "pharmaceutical composition."

As used herein, "pharmaceutically acceptable carrier" means buffers, carriers, and excipients suitable for use in contact with the tissues of human beings and animals without excessive toxicity, irritation, allergic response, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration. The use of such media and agents for pharmaceutically active substances is known in the art. In one embodiment the pharmaceutical composition is administered orally and includes an enteric coating suitable for regulating the site of absorption of the encapsulated substances within the digestive system or gut. For example, an enteric coating can include an ethylacrylate-methacrylic acid copolymer.

In one embodiment, a disclosed IL-34 antisense oligonucleotide and any pharmaceutical composition thereof may be administered by one or several routes, including topically, parenterally, orally, pulmonarily, intratracheally, intranasally, transdermally, or intraduodenally. The term parenteral as used herein includes subcutaneous injections, intrapancreatic administration, intravenous, intramuscular, intraperitoneal, intrasternal injection or infusion techniques. For example, a disclosed IL-34 antisense oligonucleotide may be administered subcutaneously to a subject. In another example, a disclosed IL-34 antisense oligonucleotide may be administered orally to a subject. In another example, a disclosed IL-34 antisense oligonucleotide may be administered directly to the gastrointestinal system, or specific regions of the gastrointestinal system (*e.g*., the ileum, colon, or rectum) via parenteral administration.

Pharmaceutical compositions containing a disclosed IL-34 antisense oligonucleotide, such as those disclosed herein, can be presented in a dosage unit form and can be prepared by any suitable method. A pharmaceutical composition should be formulated to be compatible with its intended route of administration. Useful formulations can be prepared by methods well known in the pharmaceutical art. For example, see Remington's Pharmaceutical Sciences, 18th ed. (Mack Publishing Company, 1990).

Pharmaceutical formulations, for example, are sterile. Sterilization can be accomplished, for example, by filtration through sterile filtration membranes. Where the composition is lyophilized, filter sterilization can be conducted prior to or following lyophilization and reconstitution.

### Parenteral Administration

The pharmaceutical compositions of the disclosure can be formulated for parenteral administration, *e.g*., formulated for injection via the intravenous, intramuscular, subcutaneous, intralesional, or intraperitoneal routes. The preparation of an aqueous composition, such as an aqueous pharmaceutical composition containing a disclosed IL-34 antisense oligonucleotide, will be known to those of skill in the art in light of the present disclosure. Typically, such compositions can be prepared as injectables, either as liquid solutions or suspensions; solid forms suitable for using to prepare solutions or suspensions upon the addition of a liquid prior to injection can also be prepared; and the preparations can also be emulsified.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Solutions of active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. In addition, sterile, fixed oils may be employed as a solvent or suspending medium. For this purpose any bland fixed oil can be employed including synthetic mono- or diglycerides. In addition, fatty acids such as oleic acid can be used in the preparation of injectables. The sterile injectable preparation may also be a sterile injectable solution, suspension, or emulsion in a nontoxic parenterally acceptable diluent or solvent, for example, as a solution in 1,3-butanediol. Among the acceptable vehicles and solvents that may be employed are water, Ringer's solution, U.S.P., and isotonic sodium chloride solution. In one embodiment, a disclosed IL-34 antisense oligonucleotide may be suspended in a carrier fluid comprising 1% (w/v) sodium carboxymethylcellulose and 0.1% (v/v) TWEEN™ 80. Under ordinary conditions of storage and use, these preparations contain a preservative to prevent the growth of microorganisms.

Injectable preparations, for example, sterile injectable aqueous or oleaginous suspensions may be formulated according to the known art using suitable dispersing or wetting agents and suspending agents. Generally, dispersions are prepared by incorporating the various sterilized active ingredients into a sterile vehicle which contains the basic dispersion medium and the required other ingredients from those enumerated above. Sterile injectable solutions of the disclosure may be prepared by incorporating a disclosed IL-34 antisense oligonucleotide in the required amount of the appropriate solvent with various of the other ingredients enumerated above, as required, followed by filtered sterilization. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The injectable formulations can be sterilized, for example, by filtration through a bacteria-retaining filter.

The preparation of more, or highly concentrated solutions for intramuscular injection is also contemplated. In this regard, the use of DMSO as solvent is preferred as this will result in extremely rapid penetration, delivering high concentrations of the disclosed IL-34 antisense oligonucleotide to a small area.

Suitable preservatives for use in such a solution include benzalkonium chloride, benzethonium chloride, chlorobutanol, thimerosal and the like. Suitable buffers include boric acid, sodium and potassium bicarbonate, sodium and potassium borates, sodium and potassium 10 carbonate, sodium acetate, sodium biphosphate and the like, in amounts sufficient to maintain the pH at between about pH 6 and pH 8, and for example, between about pH 7 and pH 7.5. Suitable tonicity agents are dextran 40, dextran 70, dextrose, glycerin, potassium chloride, propylene glycol, sodium chloride, and the like, such that the sodium chloride equivalent of the solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxymethylpropylcellulose, lanolin, methylcellulose , petrolatum, polyethylene glycol, polyvinyl alcohol, polyvinylpyrrolidone, carboxymethylcellulose and the like.

### Oral Administration

In some embodiments, contemplated herein are compositions suitable for oral delivery of a disclosed IL-34 antisense oligonucleotide, e.g., tablets that include an enteric coating, *e.g.,* a gastro-resistant coating, such that the compositions may deliver the IL-34 antisense oligonucleotide to, *e.g.,* the gastrointestinal tract of a patient. For example, such administration may result in a topical effect, substantially topically applying the IL-34 antisense oligonucleotide directly to an affected portion of the gastrointestinal tract of a patient. Such administration, may, in some embodiments, substantially avoid unwanted systemic absorption of the IL-34 antisense oligonucleotide.

For example, a tablet for oral administration is provided that comprises granules (*e.g.,* is at least partially formed from granules) that include a disclosed IL-34 antisense oligonucleotide, *e.g.,* an IL-34 antisense oligonucleotide, *e.g.,* an antisense oligonucleotide represented by SEQ ID NO: 1, and pharmaceutically acceptable excipients. Such a tablet may be coated with an enteric coating. Contemplated tablets may include pharmaceutically acceptable excipients such as fillers, binders, disintegrants, and/or lubricants, as well as coloring agents, release agents, coating agents, sweetening, flavoring such as wintergreen, orange, xylitol, sorbitol, fructose, and maltodextrin, and perfuming agents, preservatives and/or antioxidants.

Contemplated pharmaceutical formulations include an intra-granular phase that includes a disclosed IL-34 antisense oligonucleotide, *e.g.* an IL-34 antisense oligonucleotide, *e.g.,* an antisense oligonucleotide represented by SEQ ID NO: 1, and a pharmaceutically acceptable salt, *e.g.* an IL-34 antisense oligonucleotide, *e.g.,* an antisense oligonucleotide represented by SEQ ID NO: 1, and a pharmaceutically acceptable filler. For example, a disclosed IL-34 antisense oligonucleotide and a filler may be blended together, optionally, with other excipients, and formed into granules. In some embodiments, the intragranular phase may be formed using wet granulation, *e.g.* a liquid (*e.g.,* water) is added to the blended IL-34 antisense oligonucleotide compound and filler, and then the combination is dried, milled and/or sieved to produce granules. One of skill in the art would understand that other processes may be used to achieve an intragranular phase.

In some embodiments, contemplated formulations include an extra-granular phase, which may include one or more pharmaceutically acceptable excipients, and which may be blended with the intragranular phase to form a disclosed formulation.

A disclosed formulation may include an intragranular phase that includes a filler. Exemplary fillers include, but are not limited to, cellulose, gelatin, calcium phosphate, lactose, sucrose, glucose, mannitol, sorbitol, microcrystalline cellulose, pectin, polyacrylates, dextrose, cellulose acetate, hydroxypropylmethyl cellulose, partially pre-gelatinized starch, calcium carbonate, and others including combinations thereof.

In some embodiments, a disclosed formulation may include a intragranular phase and/or a extragranular phase that includes a binder, which may generally function to hold the ingredients of the pharmaceutical formulation together. Exemplary binders of the disclosure may include, but are not limited to, the following: starches, sugars, cellulose or modified cellulose such as hydroxypropyl cellulose, lactose, pre-gelatinized maize starch, polyvinyl pyrrolidone, hydroxypropyl cellulose, hydroxypropylmethyl cellulose, low substituted hydroxypropyl cellulose, sodium carboxymethyl cellulose, methyl cellulose, ethyl cellulose, sugar alcohols and others including combinations thereof.

Contemplated formulations, *e.g.,* that include an intragranular phase and/or an extragranular phase, may include a disintegrant such as but are not limited to, starch, cellulose, crosslinked polyvinyl pyrrolidone, sodium starch glycolate, sodium carboxymethyl cellulose, alginates, corn starch, crosmellose sodium, crosslinked carboxymethyl cellulose, low substituted hydroxypropyl cellulose, acacia, and others including combinations thereof. For example, an intragranular phase and/or an extragranular phase may include a disintegrant.

In some embodiments, a contemplated formulation includes an intra-granular phase comprising a disclosed IL-34 antisense oligonucleotide and excipients chosen from: mannitol, microcrystalline cellulose, hydroxypropylmethyl cellulose, and sodium starch glycolate or combinations thereof, and an extra-granular phase comprising one or more of: microcrystalline cellulose, sodium starch glycolate, and magnesium stearate or mixtures thereof.

In some embodiments, a contemplated formulation may include a lubricant, *e.g.* an extra-granular phase may contain a lubricant. Lubricants include but are not limited to talc, silica, fats, stearin, magnesium stearate, calcium phosphate, silicone dioxide, calcium silicate, calcium phosphate, colloidal silicon dioxide, metallic stearates, hydrogenated vegetable oil, corn starch, sodium benzoate, polyethylene glycols, sodium acetate, calcium stearate, sodium lauryl sulfate, sodium chloride, magnesium lauryl sulfate, talc, and stearic acid.

In some embodiments, the pharmaceutical formulation comprises an enteric coating. Generally, enteric coatings create a barrier for the oral medication that controls the location at which the drug is absorbed along the digestive track. Enteric coatings may include a polymer that disintegrates at different rates according to pH. Enteric coatings may include for example, cellulose acetate phthalate, methyl acrylate-methacrylic acid copolymers, cellulose acetate succinate, hydroxylpropylmethyl cellulose phthalate, methyl methacrylate-methacrylic acid copolymers, ethylacrylate-methacrylic acid copolymers, methacrylic acid copolymer type C, polyvinyl acetate-phthalate, and cellulose acetate phthalate.

Exemplary enteric coatings include Opadry® AMB, Acryl-EZE®, Eudragit® grades. In some embodiments, an enteric coating may comprise about 5% to about 10%, about 5% to about 20%, 8 to about 15%, about 8% to about 20%, about 10% to about 20%, or about 12 to about 20%, or about 18% of a contemplated tablet by weight. For example, enteric coatings may include an ethylacrylate-methacrylic acid copolymer.

For example, in a contemplated embodiment, a tablet is provided that comprises or consists essentially of about 0.5% to about 70%, *e.g.* about 0.5% to about 10%, or about 1% to about 20%, by weight of a disclosed IL-34 antisense oligonucleotide or a pharmaceutically acceptable salt thereof. Such a tablet may include for example, about 0.5% to about 60% by weight of mannitol, *e.g.* about 30% to about 50% by weight mannitol, *e.g.* about 40% by weight mannitol; and/or about 20% to about 40% by weight of microcrystalline cellulose, or about 10% to about 30% by weight of microcrystalline cellulose. For example, a disclosed tablet may comprise an intragranular phase that includes about 30% to about 60%, *e.g.* about 45% to about 65% by weight, or alternatively, about 5 to about 10% by weight of a disclosed IL-34 antisense oligonucleotide, about 30% to about 50%, or alternatively, about 5% to about 15% by weight mannitol, about 5% to about 15% microcrystalline cellulose, about 0% to about 4%, or about 1% to about 7% hydroxypropylmethylcellulose, and about 0% to about 4%, *e.g.* about 2% to about 4% sodium starch glycolate by weight.

In another contemplated embodiment, a pharmaceutical tablet formulation for oral administration of a disclosed IL-34 antisense oligonucleotide comprises an intra-granular phase, wherein the intra-granular phase includes a disclosed IL-34 antisense or a pharmaceutically acceptable salt thereof (such as a sodium salt), and a pharmaceutically acceptable filler, and which may also include an extra-granular phase, that may include a pharmaceutically acceptable excipient such as a disintegrant. The extra-granular phase may include components chosen from microcrystalline cellulose, magnesium stearate, and mixtures thereof. The pharmaceutical composition may also include an enteric coating of about 12% to 20% by weight of the tablet. For example, a pharmaceutically acceptable tablet for oral use may comprise about .5% to 10% by weight of a disclosed IL-34 antisense oligonucleotide, e.g., a disclosed IL-34 antisense oligonucleotide or a pharmaceutically acceptable salt thereof, about 30% to 50% by weight mannitol, about 10% to 30% by weight microcrystalline cellulose, and an enteric coating comprising an ethylacrylate-methacrylic acid copolymer.

In another example, a pharmaceutically acceptable tablet for oral use may comprise an intra-granular phase, comprising about 5 to about 10% by weight of a disclosed IL-34 antisense oligonucleotide, *e.g.,* a disclosed IL-34 antisense oligonucleotide or a pharmaceutically acceptable salt thereof, about 40% by weight mannitol, about 8% by weight microcrystalline cellulose, about 5% by weight hydroxypropylmethyl cellulose, and about 2% by weight sodium starch glycolate; an extra-granular phase comprising about 17% by weight microcrystalline cellulose, about 2% by weight sodium starch glycolate, about 0.4% by weight magnesium stearate; and an enteric coating over the tablet comprising an ethylacrylate-methacrylic acid copolymer.

In some embodiments the pharmaceutical composition may contain an enteric coating comprising about 13% or about 15%, 16%, 17% or 18% by weight, *e.g.,* AcyrlEZE® (see, *e.g.,* PCT Publication No. WO2010/054826).

The rate at which point the coating dissolves and the active ingredient is released is its dissolution rate. In an embodiment, a contemplated tablet may have a dissolution profile, *e.g.* when tested in a USP/EP Type 2 apparatus (paddle) at 100 rpm and 37 °C in a phosphate buffer with a pH of 7.2, of about 50% to about 100% of the IL-34 antisense oligonucleotide releasing after about 120 minutes to about 240 minutes, for example after 180 minutes. In another embodiment, a contemplated tablet may have a dissolution profile, *e.g.* when tested in a USP/EP Type 2 apparatus (paddle) at 100 rpm and 37°C in diluted HCl with a pH of 1.0, where substantially none of the IL-34 antisense oligonucleotide is released after 120 minutes. A contemplated tablet, in another embodiment, may have a dissolution profile, *e.g.* when tested in USP/EP Type 2 apparatus (paddle) at 100 rpm and 37°C in a phosphate buffer with a pH of 6.6, of about 10% to about 30%, or not more than about 50%, of the IL-34 antisense oligonucleotide releasing after 30 minutes.

Contemplated formulations, *e.g.* tablets, in some embodiments, when orally administered to the patient may result in minimal plasma concentration of the IL-34 antisense oligonucleotide in the patient. In another embodiment, disclosed formulations, when orally administered to a patient, topically deliver to the colon or rectum of a patient, *e.g.* to an affected or diseased site of a patient.

Methods provided herein may further include administering at least one other agent that is directed to treatment of diseases and disorders disclosed herein. In one embodiment, contemplated other agents may be co-administered (*e.g.,* sequentially or simultaneously).

Agents contemplated include immunosuppressive agents including glucocorticoids, cytostatics, antibodies, agents acting on immunophilins, interferons, opioids, TNF binding proteins, mycophenolate, and small biological agents. For example, contemplated immunosuppressive agents include, but are not limited to: tacrolimus, cyclosporine, pimecrolimus, sirolimus, everolimus, mycophenolic acid, fingolimod, dexamethasone, fludarabine, cyclophosphamide, methotrexate, azathioprine, leflunomide, teriflunomide, anakinra, anti-thymocyte globulin, anti-lymphocyte globulin, muromonab-CD3, afutuzumab, rituximab, teplizumab, efalizumab, daclizumab, basiliximab, adalimumab, infliximab, certolizumab pegol, natalizumab, and etanercept. Other contemplated agents include antibiotics, anti-diarrheals, laxatives, pain relievers, iron supplements, and calcium or vitamin D or B-12 supplements.

### Dosage and Frequency of Administration

Exemplary formulations include dosage forms that include or consist essentially of about 35 mg to about 500 mg of a disclosed IL-34 antisense oligonucleotide. For example, formulations that include about 35 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, or 250 mg of a disclosed IL-34 antisense oligonucleotide are contemplated herein. In one embodiment, a formulation may include about 40 mg, 80 mg, or 160 mg of a disclosed IL-34 antisense oligonucleotide. In some embodiments, a formulation may include at least 100 µg of a disclosed IL-34 antisense oligonucleotide. For example, formulations may include about 0.1 mg, 0.2 mg, 0.3 mg, 0.4 mg, 0.5 mg, 1 mg, 5 mg, 10 mg, 15 mg, 20 mg, or 25 mg of a disclosed IL-34 antisense oligonucleotide. The amount administered will depend on variables such as the type and extent of disease or indication to be treated, the overall health and size of the patient, the *in vivo* potency of the IL-34 antisense oligonucleotide, the pharmaceutical formulation, and the route of administration. The initial dosage can be increased beyond the upper level in order to rapidly achieve the desired blood-level or tissue level. Alternatively, the initial dosage can be smaller than the optimum, and the dosage may be progressively increased during the course of treatment. Human dosage can be optimized, *e.g.,* in a conventional Phase I dose escalation study designed to run from 40 mg to 160 mg. Dosing frequency can vary, depending on factors such as route of administration, dosage amount and the disease being treated. Exemplary dosing frequencies are once per day, once per week and once every two weeks. In some embodiments, dosing is once per day for 7 days.

### Diagnostic Methods

The disclosure also provides a method of diagnosing a patient with an inflammatory disease that relies upon detecting levels of IL-34 expression signal in one or more biological samples of a patient. As used herein, the term "IL-34 expression signal" can refer to any indication of IL-34 gene expression, or gene or gene product activity. IL-34 gene products include RNA (*e.g.,* mRNA), peptides, and proteins. Indices of IL-34 gene expression that can be assessed include, but are not limited to, IL-34 gene or chromatin state, IL-34 gene interaction with cellular components that regulate gene expression, IL-34 gene product expression levels (*e.g.,* IL-34 RNA expression levels, IL-34 protein expression levels), or interaction of IL-34 RNA or protein with transcriptional, translational, or post-translational processing machinery. Indices of IL-34 gene product activity include, but are not limited to, assessment of IL-34 signaling activity (*e.g.,* assessment of CSF1R activation or MAPK1/MAPK3 phosphorylation) and assessment of IL-34 receptor binding (*e.g.,* CSF1R binding).

Detection of IL-34 expression signal may be accomplished through *in vivo, in vitro,* or *ex vivo* methods. In a preferred embodiment, methods of the disclosure may be carried out *in vitro.* Methods of detecting may involve detection in blood, serum, fecal matter, tissue, or cells of a patient. Detection may be achieved by measuring IL-34 expression signal in whole tissue, tissue explants, cell cultures, dissociated cells, cell extract, or body fluids, including blood or serum. Contemplated methods of detection include assays that measure levels of IL-34 gene product expression such as Western blotting, FACS, ELISA, other quantitative binding assays, cell or tissue growth assays, Northern blots, quantitative or semi-quantitative polymerase chain reaction, medical imaging methods (*e.g.,* MRI), or immunostaining methods (*e.g.,* immunohistochemistry or immunocytochemistry).

### EXAMPLES

The invention is defined in the claims and any of the following examples that do not fall within the scope of the claims are provided for comparative purposes only.

The disclosure is further illustrated by the following examples. The examples are provided for illustrative purposes only, and are not to be construed as limiting the scope or content of the disclosure in any way.

### Example 1: IL-34 Antisense Oligonucleotides Reduce IL-34 mRNA Expression in HT-29 cells and Poly (I:C)-stimulated PBMCs

The effect of IL-34 antisense oligonucleotides on IL-34 mRNA expression in HT-29 cells was investigated. Lipofectamine 3000 (Life Technologies) was used to transfect IL-34 antisense oligonucleotides (Table 1) into HT-29 cells. HT-29 cells were cultured in McCoy's and RPMI1640 medium, respectively, supplemented with 10% FBS, and transfected with increasing concentrations of antisense oligonucleotides (0.5 µg/mL, 1 µg/mL, or 2 µg/mL) for 24h. IL-34 expression was evaluated by Real-Time PCR (RT-PCR), using β-actin as a reference.

**Table 1**

| SEQ ID | Sequence |
|---|---|
| 1 | 5'-GCTGGGTGACGCTTT-3' |
| 2 | 5'-TGGTCACTCAGTCAGG-3' |
| 3 | 5'-CCAAGATAGCGCAGCC-3' |
| 4 | 5'-GTCAAGGGCCACATCT-3' |
| 5 | 5'-AGCTGCTCAGTGTGAA-3' |
| 6 | 5'-ACAGAGGCCTAAGCA-3' |
| 7 | 5'-CTCATTCTGCGTCAAG-3' |
| 8 | 5'-TCGGGGCAGCAGAG-3' |
| 9 | 5'-GGAGTGAGCAGGTGC-3' |

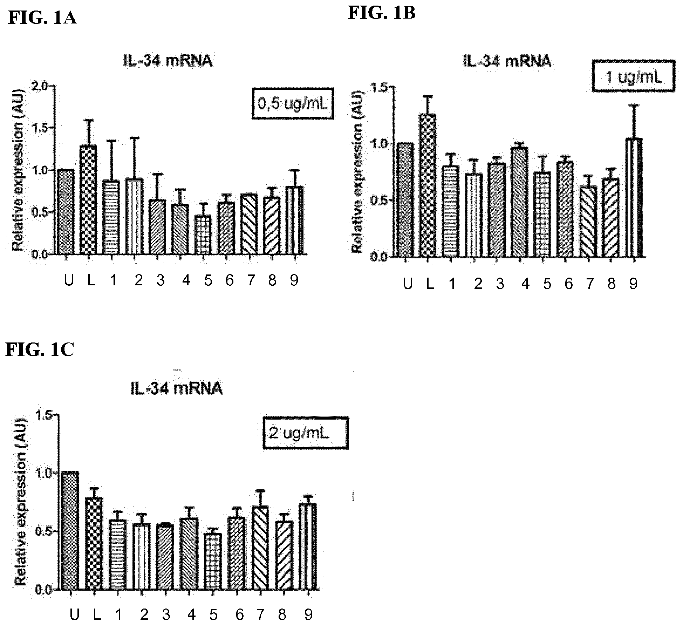

FIG. 1A shows that IL-34 mRNA expression was reduced in HT-29 cells transfected with 0.5 µg/mL of the IL-34 antisense oligonucleotide (SEQ ID NO: 1, 2, 3, 4, 5, 6, 7, 8, and 9) compared with the unstimulated control (U). FIG. 1B shows that IL-34 mRNA expression was reduced in HT-29 cells transfected with 1 µg/mL of IL-34 antisense oligonucleotide of SEQ ID NOs: 1, 2, 3, 4, 5, 6, 7, and 8). No reduction in IL-34 mRNA expression was observed in cells transfected with 1 µg/mL of the antisense oligonucleotide of SEQ ID NO: 9. Figure 1C shows that IL-34 mRNA expression was reduced in HT-29 cells transfected with 2 µg/mL of IL-34 antisense oligonucleotide (SEQ ID NO: 1, 2, 3, 4, 5, 6, 7, 8, and 9). These results are summarized in Table 2, which shows the IL-34 mRNA reduction percentage in HT-29 cells transfected with the IL-34 antisense oligonucleotides compared with the unstimulated control (U). The results demonstrate that transfection of each of the IL-34 antisense oligonucleotides tested was able to reduce IL-34 mRNA expression in HT-29 cells (with the exception of transfection of the antisense oligonucleotide of SEQ ID NO: 9 at a concentration of 1 µg/mL).

**Table 2**

| SEQ ID | 0.5 µg/mL | 1 µg/mL | 2 µg/mL |
|---|---|---|---|
| 1 | 13% | 20% | 41% |
| 2 | 11% | 27% | 45% |
| 3 | 35% | 18% | 45% |
| 4 | 41% | 4% | 40% |
| 5 | 55% | 26% | 53% |
| 6 | 39% | 16% | 39% |
| 7 | 29% | 39% | 29% |
| 8 | 33% | 32% | 42% |
| 9 | 20% | 0% | 47% |

The effects of IL-34 antisense oligonucleotides on IL-34 mRNA expression in Poly (I:C)-stimulated normal peripheral blood mononuclear cells (PBMCs) was investigated. Lipofectamine 3000 (Life Technologies) was used to transfect IL-34 antisense oligonucleotides (Table 1) into PBMCs. PBMCs were cultured in McCoy's and RPMI1640 medium, respectively, supplemented with 10% FBS, and transfected with increasing concentrations of antisense oligonucleotides (0.5 µg/mL, 1 µg/mL, or 2 µg/mL) for 24h. IL-34 expression was evaluated by RT-PCR, using β-actin as a reference. To induce IL-34 expression in PBMCs, cells were treated with 8 mg/mL Poly (I:C) for 6 hours after transfection.

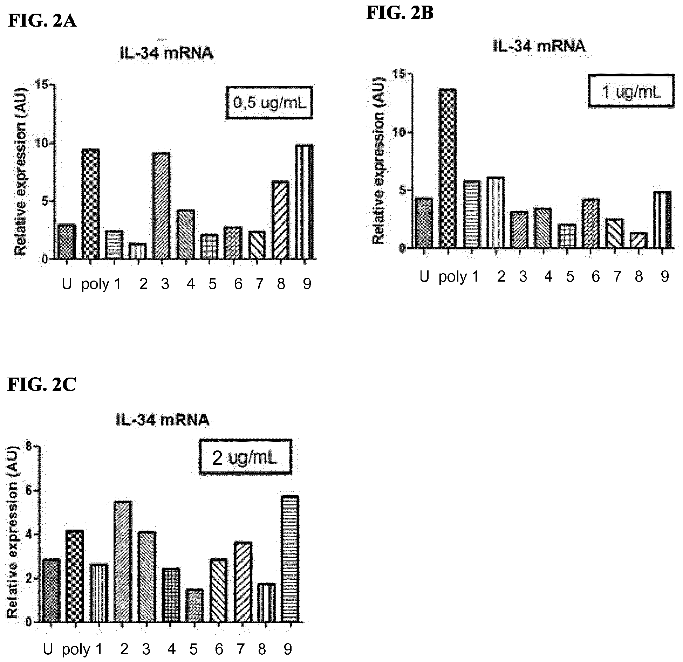

FIG. 2A shows that IL-34 mRNA expression was reduced in Poly (I:C)-stimulated PBMCs transfected with 0.5 µg/mL of the IL-34 antisense oligonucleotide of SEQ ID NO: 1, 2, 3, 4, 5, 6, 7, or 8, compared to untransfected Poly (I:C)-stimulated PBMCs (poly). No reduction was observed in IL-34 mRNA expression levels in Poly (I:C)-stimulated PBMCs transfected with 0.5 µg/mL of the IL-34 antisense oligonucleotide of SEQ ID NO: 9. FIG. 2B shows that IL-34 mRNA expression was reduced in Poly (I:C)-stimulated PBMCs transfected with 1 µg/mL of the IL-34 antisense oligonucleotide (SEQ ID NO: 1, 2, 3, 4, 5, 6, 7, 8, and 9). FIG. 2C shows that IL-34 mRNA expression was reduced in Poly (I:C)-stimulated PBMCs transfected with 2 µg/mL of the IL-34 antisense oligonucleotide of SEQ ID NO: 1, 3, 4, 5, 6, 7, and 8. No reduction was observed in IL-34 mRNA expression levels in Poly (I:C)-stimulated PBMCs transfected with 2 µg/mL of the IL-34 antisense oligonucleotide of SEQ ID NO: 2 or 9. The results of Poly (I:C)-stimulated PBMC transfection with IL-34 antisense oligonucleotides at concentrations of 0.5 µg/mL and 1 µg/mL are summarized in Table 3, which shows the IL-34 mRNA reduction percentage in Poly (I:C)-stimulated PBMCs transfected with the IL-34 antisense oligonucleotides compared with the control (poly). The results demonstrate that transfection of each of the IL-34 antisense oligonucleotides tested was able to reduce IL-34 mRNA expression in Poly (I:C)-stimulated PBMCs at almost all of the transfection concentrations tested. Table 3

| SEQ ID | 0.5 µg/mL | 1 µg/mL |
|---|---|---|
| 1 | 75% | 48% |
| 2 | 86% | 12% |
| 3 | 3% | 39% |
| 4 | 56% | 58% |
| 5 | 79% | 75% |
| 6 | 71% | 51% |
| 7 | 76% | 47% |
| 8 | 29% | 74% |
| 9 | 0% | 13% |

### Example 2: IL-34 Expression is Increased in Colorectal Cancer (CRC) Tumoral Tissue

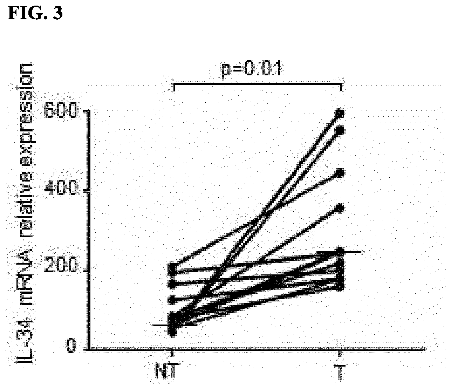

In order to determine whether increased IL-34 expression is associated with colorectal tumoral tissue, paired colonic explants were harvested from non-tumoral (NT) and tumoral (T) areas of 12 patients with sporadic CRC. RT-PCR was performed to analyze the level of IL-34 mRNA expression in the explants. IL-34 mRNA levels were normalized using β-actin mRNA levels as a reference. The results of the RT-PCR analysis are shown in Fig. 3. Each point on the graph of FIG. 3 represents the IL-34 mRNA expression level in a single patient, with lines connecting the data points collected from tumoral and non-tumoral tissue from the same patient. The horizontal bars in the NT and T columns indicate the median IL-34 mRNA expression value in each group. The results demonstrate that IL-34 expression was increased in all individual tumoral tissue samples compared to non-tumoral tissue samples from the same patient. The results also demonstrate that the overall medial level of IL-34 mRNA was significantly increased in tumoral tissue compared to non-tumoral tissue from CRC patients (FIG. 3; NT v. T, p = 0.01).

### Example 3: IL-34 Expression is Increased in Sporadic CRC Tumoral Tissue

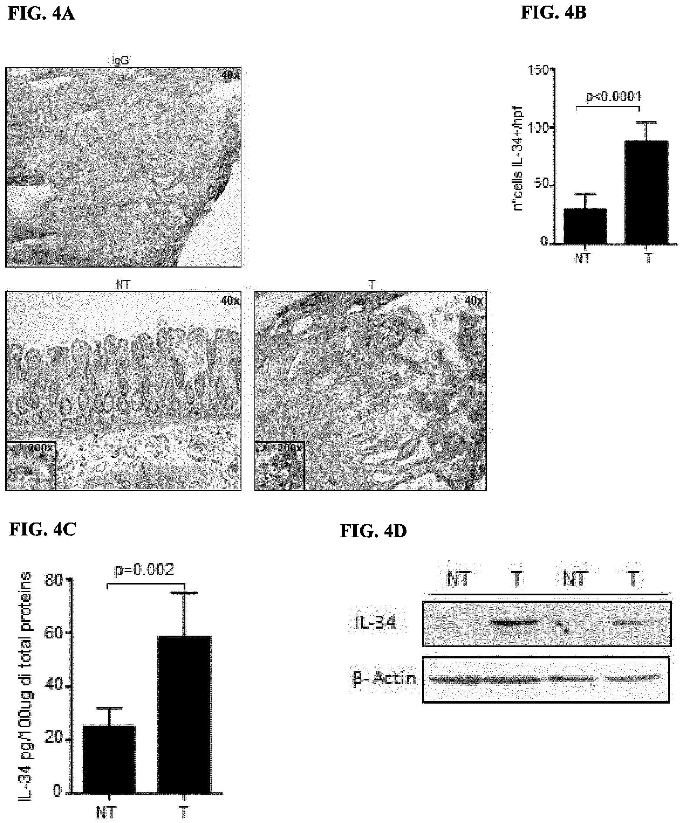

To determine if IL-34 protein expression is increased in sporadic CRC tumoral tissue, colonic tissue samples of sporadic CRC patients were harvested from non-tumoral (NT) and tumoral (T) areas of colonic tissue and analyzed by immunohistochemistry. Frozen tissue samples were exposed to either isotype control antibody (IgG) (FIG. 4A, upper panel) or IL-34 antibody (FIG. 4A, lower panels). Tissue exposed to isotype control and non-tumoral tissue exposed to an IL-34 detection antibody displayed little or no visible IL-34 antibody signal. Tumoral tissue exposed to IL-34 detection antibody showed a clear increase in IL-34 antibody signal compared to non-tumoral tissue and isotype control samples (FIG. 4A). Representative photomicrographs show IL-34 detection antibody and isotype control staining at 40x magnification, with 200x images inset (FIG. 4A). Quantification of the number of IL-34-positive cells in tumoral (T) tissue compared to non-tumoral (NT) tissue demonstrated a significant increase in the number of IL-34-positive cells per high power field (hpf; *i.e.,* 200x)(FIG. 4B; n = 8 patients; NT v. T, p < 0.0001; bars represent SEM).

ELISA was also performed on colonic explant tissue taken from the non-tumoral (NT) and tumoral (T) areas of 8 patients with sporadic CRC to measure IL-34 protein levels (FIG 4C). Quantification of the IL-34 protein concentration in tumoral (T) tissue compared to non-tumoral (NT) tissue demonstrated a significant increase in IL-34 protein concentration in tumoral tissue (FIG. 4C; n = 8 patients; NT v. T, p = 0.002; bars represent SEM).

Western blotting was also performed to determine whether IL-34 protein levels are increased in tumoral colonic tissue of sporadic CRC patients. IL-34 detection antibody was used to probe Western blots containing tissue extract from non-tumoral (NT) and tumoral (T) areas of 5 patients with sporadic CRC. Blots were also probed with a β-actin antibody to provide a control. FIG. 4D shows a representative Western blot of tumoral and non-tumoral colonic tissue extracts from 2 sporadic CRC patients probed with IL-34 antibody and β-actin control antibody. Analysis of Western blots probed with IL-34 antibody showed a clear increase in the level of IL-34 antibody signal in tumoral colonic tissue compared to non-tumoral colonic tissue from sporadic CRC patients.

These results demonstrate that IL-34 protein levels was significantly increased in tumoral colonic tissue of patients with sporadic CRC, compared to non-tumoral colonic tissue.

### Example 4: IL-34 Induces Cell Proliferation Through an ERK1/2-dependent Pathway

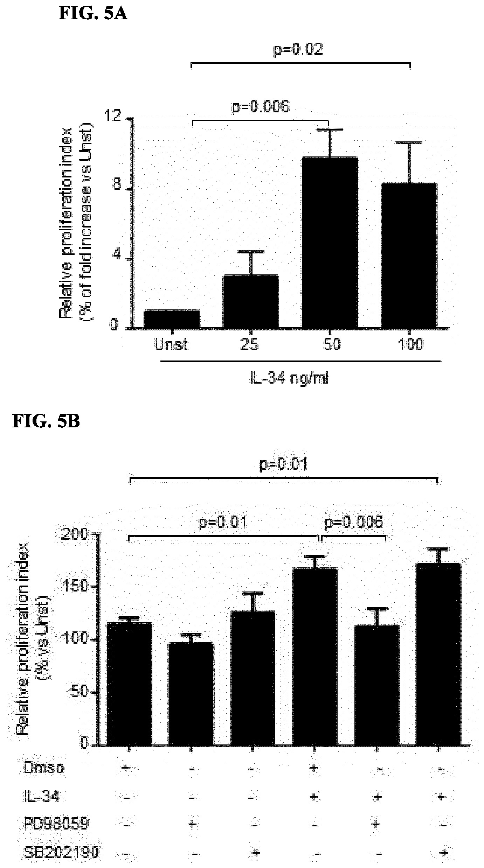

To determine whether IL-34 stimulates cell proliferation, serum-starved DLD-1 cancer cells were left unstimulated (Unst) or were stimulated with increasing doses of recombinant human IL-34 protein (25, 50, or 100 ng/ml) for 48 hours. DLD-1 cell proliferation was evaluated with carboxyfluorescein succinimidyl ester (CFSE) staining by flow cytometry. Stimulation of DLD-1 cells with 50 ng/ml and 100 ng/ml both resulted in a significant increase in cell proliferation compared to unstimulated control samples (FIG 5A; n = 4; 50 ng/ml v. Unst, p = 0.006; 100 ng/ml v. Unst, p = 0.02; bars represent SEM). These results demonstrate that IL-34 stimulated significant increases in cell proliferation of serum-starved DLD-1 cells.

To determine whether IL-34 stimulates cell proliferation via an ERK1/2 or p38-dependent pathway, serum-starved DLD-1 cells were pre-incubated with specific inhibitors of either ERK1/2 or p38 (PD98059 or SB202190, respectively) or with vehicle DMSO for 1 hour and then stimulated with recombinant human IL-34 protein at a concentration of 50 ng/mL for 48 hours. DLD-1 cell proliferation was evaluated with CFSE staining by flow cytometry. Stimulation of serum-starved DLD-1 cells with IL-34 protein resulted in a significant increase in cell proliferation compared to proliferation of cells exposed to vehicle alone (FIG. 5B; n = 4; IL-34 + DMSO v. DMSO, p = 0.01; bars represent SEM). IL-34 stimulation of cells pre-incubated with the p38 blocker SB202190 resulted in a significant increase in cell proliferation compared to unstimulated controls pre-incubated with vehicle (comparable to the increase in cell proliferation of cells exposed to IL-34 alone) but not compared to cells that were pre-incubated with vehicle and stimulated with IL-34 alone (FIG. 5B, DMSO v. IL-34 + SB202190, p = 0.01; bars represent SEM). However, proliferation of DLD-1 cells stimulated with IL-34 and pre-incubated with vehicle was significantly greater than proliferation of DLD-1 cells pre-incubated with the ERK1/2 blocker PD98059 and subsequently stimulated with IL-34 protein (FIG. 5B; DMSO + IL-34 v. PD98059 + IL-34, p = 0.006; bars represent SEM). These results demonstrate that IL-34 protein was able to stimulate proliferation of cells through an ERK1/2-dependent pathway.

### Example 5: IL-34 Increases TNF-α-Induced Apoptosis in a CRC Cell Line

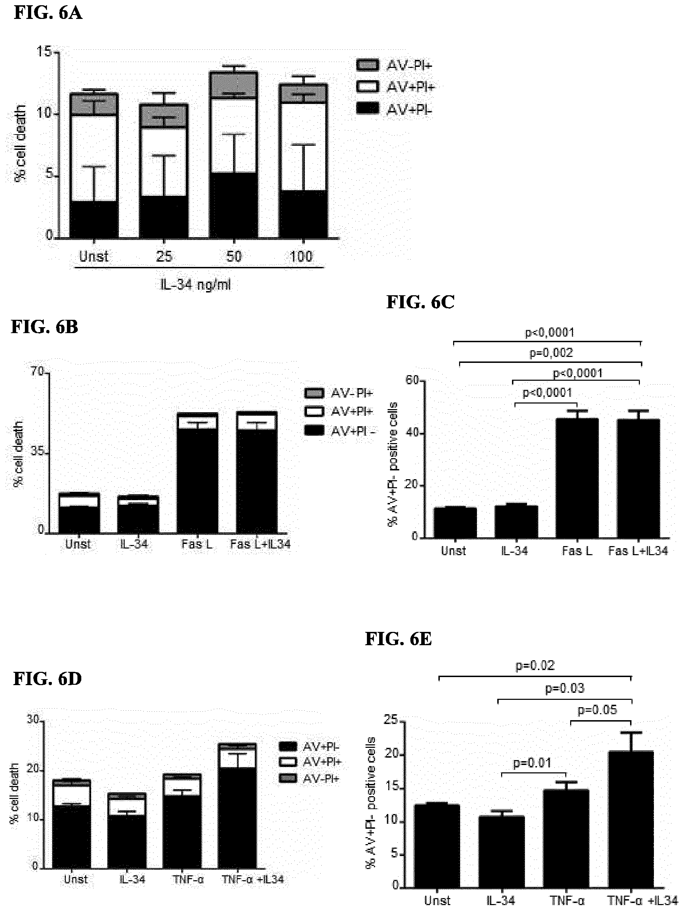

To determine if IL-34 stimulates apoptosis of CRC cells, serum-starved DLD-1 cells were either left unstimulated (Unst) or stimulated with increasing doses of recombinant human IL-34 (25 ng/ml, 50 ng/ml, 100 ng/ml) for 48 hours. Cells were analyzed by flow cytometry for PI and AV to determine the percent of cell death in each sample. No significant increase in cell death was observed in IL-34-stimulated samples compared to unstimulated controls (FIG. 6A; n = 4; bars represent mean ± SEM).

To determine if IL-34 potentiates Fas ligand-mediated cell death, serum-starved DLD-1 cells were either left unstimulated (Unst) or stimulated with recombinant human IL-34 at a concentration of 50 ng/ml and/or Fas Ligand (Fas L) at a concentration of 200 ng/ml for 48 hours. Cells were analyzed by flow cytometry for PI and/or AV signal to determine the percent of cell death in each sample. FIG. 6B shows the percent of cell death as assessed by AV⁺PI⁺, AV⁺PI⁻, and AV⁻PI⁺ staining. FIG 6C shows the percent of cell death as assessed by AV⁺PI⁻ staining alone. Stimulation of DLD-1 cells with IL-34 and FasL did not result in a significant increase in cell death compared to FasL stimulation alone (FIG. 6B and 6C; n = 4; bars represent mean ± SEM).

To determine if IL-34 potentiates TNFα-mediated cell death, serum-starved DLD-1 cells were either left unstimulated (Unst) or stimulated with recombinant human IL-34 at a concentration of 50 ng/ml and/or TNFα at a concentration of 100 ng/ml for 48 hours. Cells were analyzed by flow cytometry for PI and/or AV signal to determine the percent of cell death in each sample. FIG. 6D shows the percent of cell death as assessed by AV⁺PI⁺, AV⁺PI⁻, and AV⁻PI⁺ staining. FIG 6E shows the percent of cell death as assessed by AV⁺PI⁻ staining alone. Stimulation of DLD-1 cells with IL-34 and TNFα resulted in a significant increase in cell death compared to either IL-34 or TNFα stimulation alone (FIG. 6D and 6E; n = 4; IL-34 v. IL34 + TNFα, p = 0.03; TNFα v. IL34 + TNFα, p = 0.05; bars represent mean ± SEM). These results demonstrate that IL-34 was able to potentiate the amount of cell death in CRC cells caused by stimulation of TNFα alone.

### Example 6: IL-34 Receptors are Increased in Human Sporadic CRC

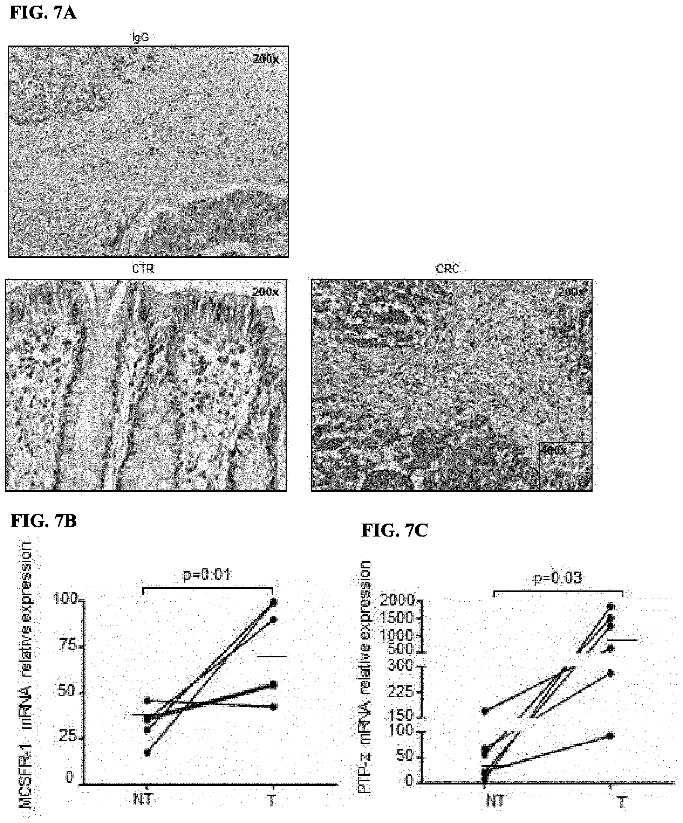

To determine if the expression of IL-34 receptor protein is increased in human sporadic CRC, colonic tissue samples were harvested from 3 control patients (CTR) and 3 patients with sporadic CRC, sectioned, and stained with antibody specific for the IL-34 receptor macrophage colony-stimulating factor-1 receptor (M-CSFR-1) or isotype control IgG. Photomicrographs of control isotype IgG-stained tissue sections showed no significant signal (FIG. 7A, top panel). M-CSFR-1-stained paraffin-embedded tissue sections of control patients (FIG. 7A, lower left panel) showed lower amounts of M-CSFR-1 signal compared to tissue sections from sporadic CRC patients (FIG. 7A, lower right panel; photomicrographs are representative of all samples; original magnification, 200x; inset, 400x).

To determine if the expression of IL-34 receptor mRNA is increased in human sporadic CRC, colonic explants harvested from the non-tumoral (NT) and tumoral (T) colonic tissue from 6 patients with sporadic CRC were analyzed for M-CSFR-1 mRNA expression by RT-PCR. Colonic tissue was also analyzed for expression of mRNA encoding an alternate IL-34 receptor PTP-ζ. Results were normalized using β-actin mRNA levels as a reference. FIG. 7B shows normalized M-CSFR-1 mRNA levels in NT and T colonic tissue from 6 individuals with sporadic CRC. FIG. 7C shows normalized PTP-ζ mRNA levels in NT and T colonic tissue from 6 individuals with sporadic CRC. NT and T data points on each graph from the same patient are connected by lines. Horizontal bars in each column of FIG. 7B and 7C represent the median M-CSFR-1 or PTP-ζ mRNA values, respectively, in each of the NT and T groups. FIG 7B indicates that levels of M-CSFR-1 mRNA were higher in tumoral colonic tissue compared to non-tumoral colonic tissue in each individual, and that median levels of M-CSFR-1 were significantly higher in tumoral colonic tissue compared to non-tumoral colonic tissue (FIG. 7B, p = 0.01). FIG 7C indicates that levels of PTP-ζ mRNA were higher in tumoral colonic tissue compared to non-tumoral colonic tissue in each individual, and that median levels of PTP-ζ were significantly higher in tumoral colonic tissue compared to non-tumoral colonic tissue (FIG. 7C, p = 0.03).

These results demonstrate that both mRNA and protein levels of IL-34 receptors are increased in tumoral tissue of sporadic CRC patients.

### Example 7: Macrophage colony-stimulating factor -1 Does Not Affect Cell Growth or Death of CRC Cells

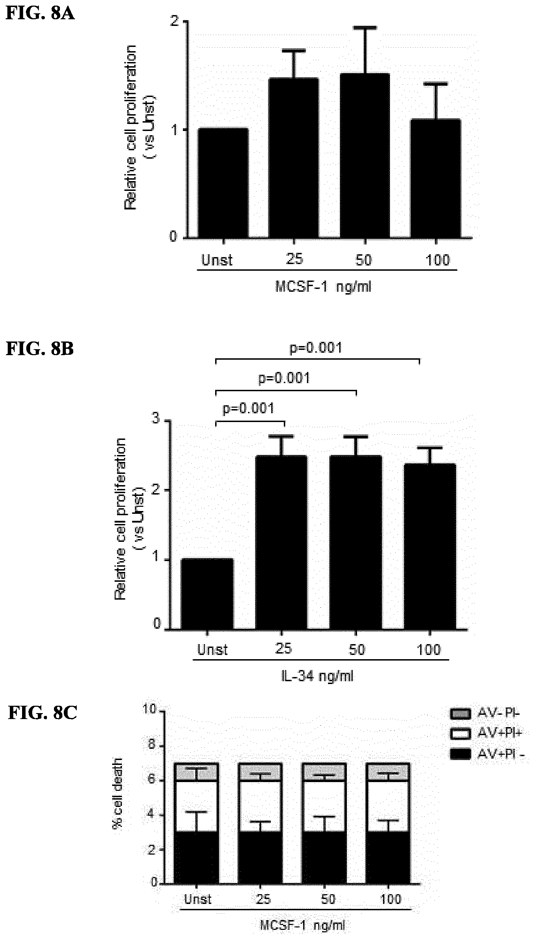

To determine whether the M-CSFR-1 ligand Macrophage colony-stimulating factor -1 (MCSF-1) affects CRC cell growth, serum-starved DLD-1 cells were either left unstimulated (Unst) or stimulated with increasing doses of recombinant human MCSF-1 (25 ng/ml, 50 ng/ml, 100 ng/ml). Cell proliferation of DLD-1 cells was analyzed by counting 5-bromo-2'-deoxyuridine (BrdU)-positive cells. No significant increase in cell proliferation was detected following stimulation with MCSF-1 (FIG. 8A, n = 11; bars represent mean ± SEM).

Serum-starved DLD-1 cells were also either left unstimulated (Unst) or stimulated with increasing doses of recombinant human IL-34 (25 ng/ml, 50 ng/ml, 100 ng/ml). Cell proliferation of DLD-1 cells was analyzed by counting BrdU-positive cells. IL-34 stimulation at all concentrations resulted in a significant increase in cell proliferation compared to unstimulated controls (FIG. 8B, n = 7; Unst v. 25 ng/ml, p = 0.001; Unst v. 50 ng/ml, p = 0.001; Unst v. 100 ng/ml, p = 0.001; bars represent mean ± SEM).

To determine whether the M-CSFR-1 ligand Macrophage colony-stimulating factor -1 (MCSF-1) affects CRC cell death, serum-starved DLD-1 cells were either left unstimulated (Unst) or stimulated with increasing doses of recombinant human MCSF-1 (25 ng/ml, 50 ng/ml, 100 ng/ml). Cells were analyzed by flow cytometry for PI and/or AV signal to determine the percent of cell death in each sample. No significant increase in cell death was detected following stimulation with MCSF-1 (FIG. 8C, n = 6; bars represent mean ± SEM).

These results demonstrate that, unlike IL-34, the M-CSFR-1 ligand MCSF-1 has no appreciable effect on cell proliferation or growth of CRC cells.

### Example 8: The HT-29 CRC Cell Lines Expresses IL-34 at High Levels

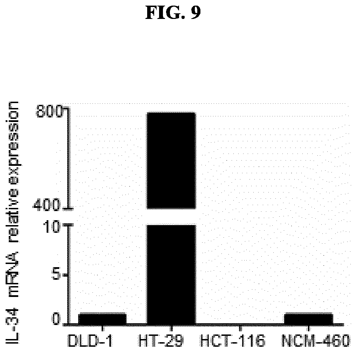

To evaluate IL-34 expression in human CRC cell lines, IL-34 mRNA expression was analyzed by RT-PCR in the DLD-1, HT-29, and HCT-116 human CRC cell lines, and in the NCM-460 normal intestinal epithelial cell line. IL-34 mRNA expression levels were normalized using β-actin mRNA levels as a reference. As shown in FIG. 9, IL-34 mRNA expression was abundant in HT-29 cells.

### Example 9: An IL-34 Antisense Oligonucleotide Knocks Down Levels of IL-34 and Inhibits CRC Cell Proliferation Through an ERK1/2-Dependent Mechanism

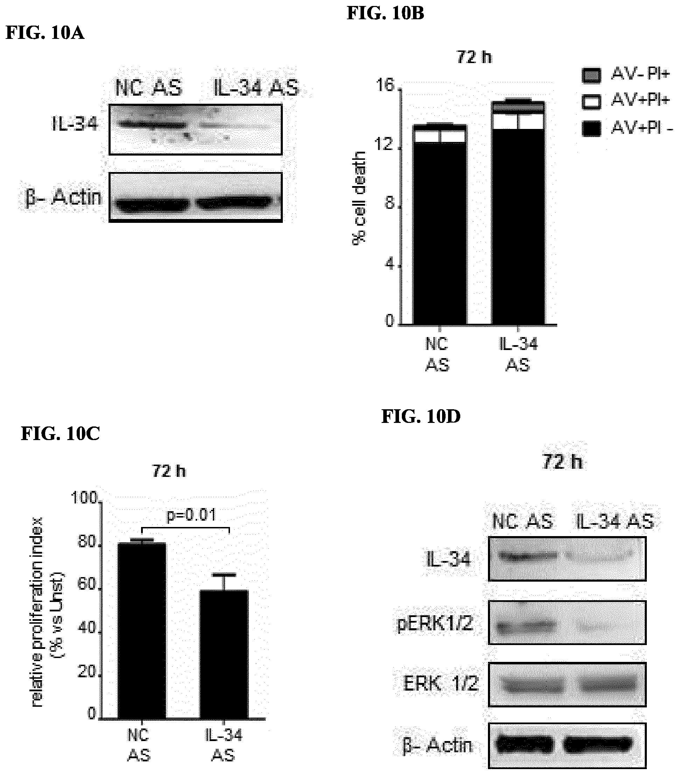

To determine whether IL-34 antisense oligonucleotides are capable of downregulating IL-34 protein expression in the DLD-1 CRC cell line, DLD-1 cells were transfected with a negative control antisense oligonucleotide (NC AS) or an IL-34 antisense Hconcentration of 2 µg/ml for 48 hours. Protein was extracted from DLD-1 cells, and Western blots were probed with an IL-34 antibody or a β-actin antibody to provide a control. Transfection of the IL-34 antisense oligonucleotide resulted in an appreciable decrease in IL-34 antibody signal (FIG. 10A; representative image from one of four experiments shown). This result demonstrates that an IL-34 antisense oligonucleotide was able to appreciably knock down IL-34 levels in a CRC cell line.

To determine whether decreased IL-34 levels in DLD-1 cells results in increased cell death, DLD-1 cells were transfected with a negative control antisense oligonucleotide (NC AS) or an IL-34 antisense oligonucleotide (IL-34 AS; IL-34 antisense oligonucleotide of SEQ ID NO: 5) at a concentration of 2 µg/ml for 72 hours. The percentage of cell death was assessed by flow cytometry analysis as percent of AV-positive and/or PI-positive cells. No significant increase in DLD-1 cell death was observed in IL-34 AS samples compared to NC AS samples (FIG. 10B; n = 4; data is expressed as mean ± SEM). These results demonstrate that IL-34 knockdown had no significant effect on cell death in the DLD-1 cell line.

To determine whether decreased IL-34 levels in DLD-1 cells results in increased cell proliferation, DLD-1 cells were transfected with a negative control antisense oligonucleotide (NC AS) or an IL-34 antisense oligonucleotide (IL-34 AS; IL-34 antisense oligonucleotide of SEQ ID NO: 5) at a concentration of 2 µg/ml for 72 hours. Cell proliferation was evaluated by flow cytometry following staining with CFSE. Transfection with IL-34 AS resulted in a significant decrease in DLD-1 cell proliferation compared to NC AS transfection (FIG. 10C; n = 4; NC AS v. IL-34 AS, p = 0.01; data is expressed as mean ± SEM). This result demonstrates that IL-34 knockdown significantly decreased DLD-1 cell proliferation.

To determine whether IL-34 knockdown affects expression of ERK1/2 protein, DLD-1 cells were transfected with either NC AS or IL-34 AS (IL-34 antisense oligonucleotide of SEQ ID NO: 5) at a concentration of 2 µg/ml for 72 hours. Protein was extracted from DLD-1 cells, and Western blots were probed for IL-34, total ERK1/2 (ERK1/2), phosphorylated ERK1/2 (pERK1/2), or β-actin. Transfection with IL-34 AS resulted in an appreciable knockdown in levels of IL-34 as well as pERK1/2, but not total ERK1/2 (FIG 10D; image representative of 4 experiments). These results demonstrate that IL-34 knockdown also caused a decrease in levels of pERK1/2 in DLD-1 cells. These results also suggest that IL-34 expression was necessary to maintain higher levels of pERK1/2, and that IL-34 effects on CRC cell proliferation may occur through an ERK1/2-dependent pathway.

### Example 10: IL-34 and Its Receptor are Expressed in Colitis-Associated Colon Cancer Tumors

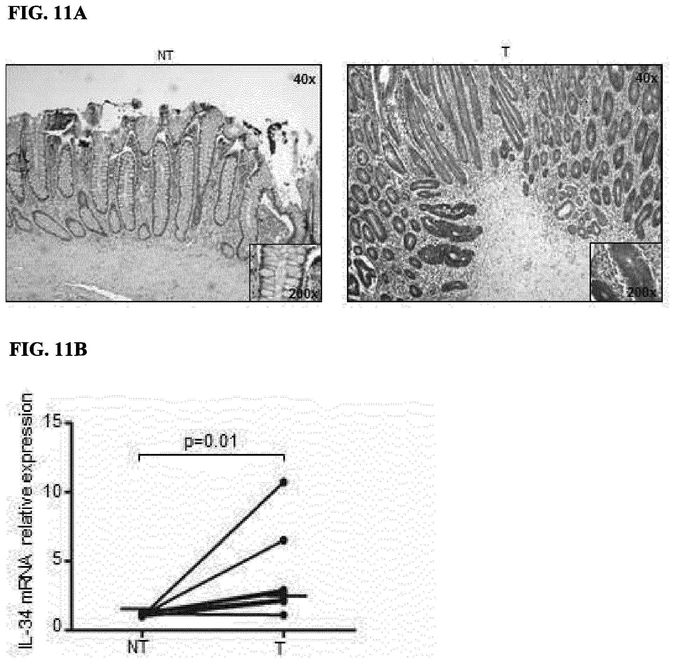

To determine whether M-CSFR-1 is expressed in colitis-associated colon cancer (CAC) tumor tissue, tumoral and non-tumoral colonic tissue harvested from three CAC patients was sectioned, embedded in paraffin, and stained with an M-CSFR-1 antibody. M-CSFR-1 signal appeared more abundant in colonic tumoral tissue, compared to colonic non-tumoral tissue (FIG. 11A; representative micrographs taken at 40x magnification; insets at 200x magnification).

To determine whether IL-34 is expressed in CAC tumor tissue, tumoral and non-tumoral colonic tissue was harvested from wild-type C57BL/6J mice in which CAC was induced by exposure to AOM and DSS. Paired explants were harvested from non-tumoral and tumoral areas of colonic tissue from 7 mice sacrificed at day 84 after AOM+DSS treatment were analyzed for IL-34 mRNA expression by RT- PCR. IL-34 mRNA levels were normalized to β-actin mRNA levels. FIG. 11B shows normalized IL-34 mRNA levels in non-tumoral (NT) and tumoral (T) colonic tissue from 7 mice. NT and T data points from the same animal are connected by lines. Horizontal bars in each column represent median IL-34 mRNA values in the NT and T groups. Median levels of IL-34 were significantly higher in tumoral colonic tissue compared to non-tumoral colonic tissue from CAC mice (FIG. 11B, p = 0.01).

These results demonstrate that levels of both IL-34 and its receptor, M-CSFR-1, are increased in CAC tumoral tissue.

### Example 11: IL-34 Expression is Elevated in Fibro-Stricturing Crohn's Disease

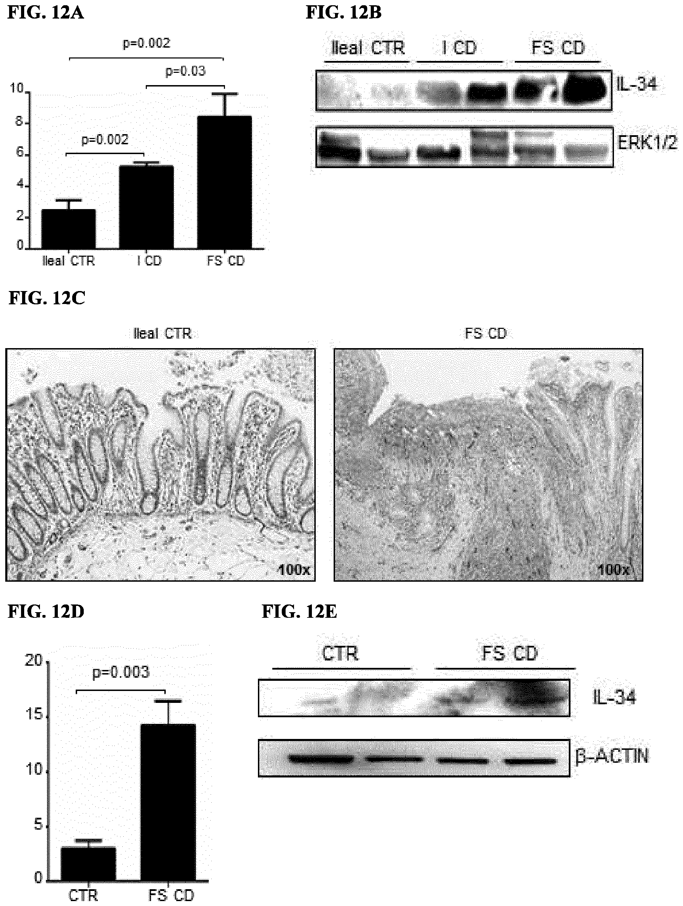

To evaluate whether IL-34 RNA expression is elevated in the intestinal tissue of individuals suffering from fibro-stricturing Crohn's disease (FS CD), ileal biopsies were harvested from 5 control patients (Ileal CTR) not suffering from inflammatory Crohn's Disease (I CD) or FS CD, 5 patients with I CD, and 5 patients with FS CD. The level of IL-34 mRNA expression in each biopsy was evaluated by RT-PCR. IL-34 mRNA levels were normalized using β-actin mRNA expression signal. IL-34 mRNA expression levels were significantly higher in I CD and FS CD samples compared to control samples (FIG. 12A; I CD v. Ileal CTR, p = 0.002; FS CD v. Ileal CTR, p = 0.03; data are expressed as mean ± SEM).

To evaluate whether IL-34 protein levels are increased elevated in the intestinal tissue of individuals suffering from FS CD, ileal biopsies were harvested from control patients (Ileal CTR) not suffering from I CD or FS CD, patients with I CD, and patients with FS CD. Protein extracts of biopsied tissue were analysed by Western blot analysis, using antibodies against IL-34 and ERK1/2 as a loading control. IL-34 protein signal appeared higher in I CD and FS CD samples compared to control samples (FIG. 12B; representative blot showing results from probe of extracts from 2 Ileal CTR patients, 2 I CD patients, and 2 FS CD patients).

To further analyze whether IL-34 protein levels are elevated in FS CD, paraffin-embedded sections of surgical intestinal samples harvested from 1 normal control (Ileal CTR) and 1 patient suffering from FS CD were stained using an IL-34 detection antibody. Analysis of the stained sections revealed the presence of IL-34-positive cells in both the lamina propria and stromal compartments of the intestine of FS CD patients. The number of IL-34-positive cells appeared higher in tissue sections from FS CD patients than comparable sections from control individuals (FIG. 12C; photomicrographs at 100x magnification are representative of images from 3 Ileal CTR individuals and 3 FS CD patients).

To determine whether IL-34 mRNA and protein expression is elevated in intestinal fibroblasts of patients with FS CD, intestinal fibroblasts were collected from 5 control patients (CTR) and 5 FS CD patients. IL-34 RNA mRNA expression in fibroblasts was evaluated RT-PCR. IL-34 mRNA expression levels were normalized using β-actin mRNA expression signal. RT-PCR analysis demonstrated that IL-34 mRNA expression levels were significantly elevated in intestinal fibroblasts of FS CD patients compared to CTR patients (FIG. 12D; CTR v. FS CD, p = 0.003; data is represented as the mean ± SEM). Total protein extracts from intestinal fibroblasts of CTR and FS CD patients were analysed by Western blot. Blots of fibroblast extracts were probed with antibodies against IL-34 and β-actin (loading control). IL-34 detection signal appeared elevated in intestinal fibroblast extracts of FS CD patients compared to samples from control patients (FIG. 12E; representative image showing probed extracts from 2 CTR and 2 FS CD patients).

These results demonstrate that IL-34 mRNA and protein expression levels were elevated in ileal tissue and in individual intestinal fibroblast cells of patients suffering from FS CD compared to IL-34 mRNA and protein expression levels in healthy patients. These results also demonstrate that IL-34 mRNA and protein expression levels were elevated in ileal tissue of patients suffering from I CD compared to IL-34 mRNA and protein expression levels in healthy patients.

### Example 12: Expression of the IL-34 Receptor M-CSFR-1 is Elevated in Inflammatory Crohn's Disease and Fibro-Sticturing Crohn's Disease

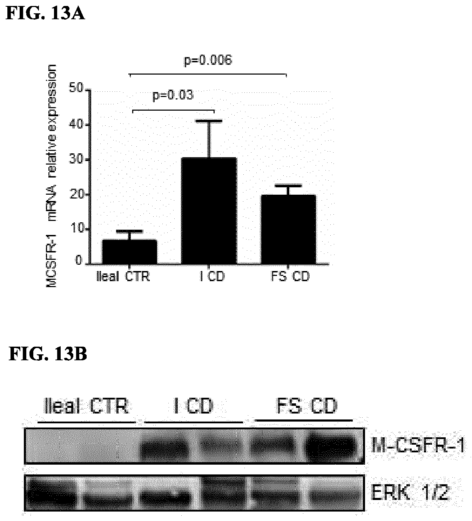

To evaluate whether expression of the IL-34 receptor, M-CSFR-1, is elevated in FS CD, ileal biopsies were harvested from 5 normal control patients (Ileal CTR), 5 patients suffering from I CD, and 5 patients with FS CD. M-CSFR-1 mRNA levels were evaluated in each sample using RT-PCR. M-CSFR-1 mRNA levels were normalized using β-actin mRNA expression signal. M-CSFR-1 mRNA expression levels were significantly elevated in I CD and FS CD samples compared to control samples (FIG. 13A; Ileal CTR v. I CD, p = 0.03; Ileal CTR v. FS CD, p = 0.006; data is expressed as mean ± SEM). Protein extracts of ileal biopsy tissue from control patients (Ileal CTR), patients suffering from I CD, and patients with FS CD were analyzed by Western blot to evaluate levels of M-CSFR-1 protein expression in FS CD. Blots of ileal biopsy tissue extracts were probed with antibodies against M-CSFR-1 and ERK1/2 (loading control). M-CSFR-1 detection signal appeared elevated in extracts of I CD and FS CD patients compared to samples from control patients (FIG. 13B; representative image showing probed extracts from 2 Ileal CTR, 2 I CD, and 2 FS CD patients).

These results demonstrate that protein and mRNA expression levels of the IL-34 receptor, M-CSFR-1, are elevated in the intestinal tissue of I CD and FS CD patients.

### Example 13: IL-34 Stimulates Collagen Production in Normal Fibroblasts

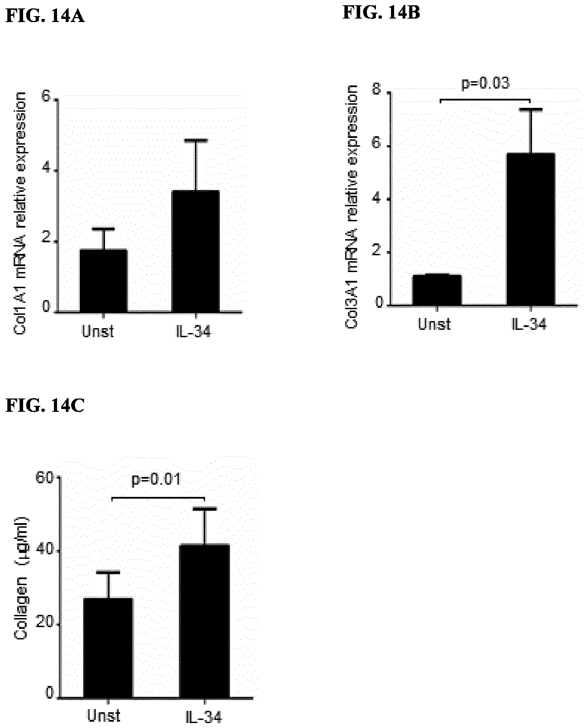

To evaluate whether exposure to IL-34 affects collagen production, normal fibroblasts were isolated from controls patients and were either left untreated (Unst) or exposed to recombinant human IL-34 protein at a concentration of 50 ng/ml for 6-48 hours. After 6 hours, Collagen Type 1 Alpha 1 chain (Col1A1; FIG 14A) and Collagen Type III Alpha 1 chain (Col3A1; FIG. 14B) mRNA levels were evaluated by RT-PCR. Col1A1 and Col3A1 mRNA levels were normalized relative to β-actin mRNA expression signal. Col1A1 mRNA expression levels were elevated in IL-34-treated samples compared to untreated samples (FIG. 14A; n = 4; data is expressed as mean ± SEM). Col3A1 mRNA expression levels were significantly elevated in IL-34-treated samples compared to untreated samples (FIG. 14B; Unst v. IL-34, p = 0.03; n = 4; data is expressed as mean ± SEM). After 48 hours, collagen secretion was evaluated in cell supernatants by Sircol assay. At 48 hours, collagen levels were significantly elevated in IL-34-treated samples compared to untreated samples (FIG. 14C; Unst v. IL-34, p = 0.01; n = 4; data is expressed as mean ± SEM). These results demonstrate that IL-34 stimulated collagen expression in fibroblast cells.

## Claims

1. An IL-34 antisense oligonucleotide comprising the nucleotide sequence 5'-AGCTGCTCAGTGTGAA-3' (SEQ ID NO:5).

2. The IL-34 antisense oligonucleotide of claim 1, wherein at least one nucleoside linkage of the sequence is selected from the group consisting of a phosphorothioate linkage, a phosphorodithioate linkage, a phosphotriester linkage, an alkylphosphonate linkage, an aminoalkylphosphotriester linkage, an alkylene phosphonate linkage, a phosphinate linkage, a phosphoramidate linkage, and an aminoalkylphosphoramidate linkage, a thiophosphoramidate linkage, thionoalkylphosphonate linkage, a thionoalkylphosphotriester linkage, a thiophosphate linkage, a selenophosphate linkage, and a boranophosphate linkage, preferably wherein at least one internucleoside linkage of the sequence is a phosphorothioate linkage; or preferably wherein all internucleoside linkages of the sequence are phosphorothioate linkages.

3. The IL-34 antisense oligonucleotide of any one of claims 1 or 2 wherein the antisense oligonucleotide is from 20 to 30, 30 to 40, or 22 to 40 nucleotides in length.

4. A pharmaceutically acceptable composition comprising the IL-34 antisense oligonucleotide of any one of claims 1-3 and a pharmaceutically acceptable carrier.

5. The pharmaceutical composition of claim 4, wherein the pharmaceutical composition is suitable for topical, parenteral, oral, pulmonary, intratracheal, intranasal, transdermal, or intraduodenal administration, preferably wherein the pharmaceutical composition is suitable for oral administration.

6. An IL-34 antisense oligonucleotide of any one of claims 1-3 for use as a medicament.

7. An IL-34 antisense oligonucleotide of any one of claims 1-3 for use in the treatment of an inflammatory disease.

8. The IL-34 antisense oligonucleotide for use as claimed in claim 7, wherein said inflammatory disease is associated with altered IL-34 expression.

9. The IL-34 antisense oligonucleotide for use as claimed in claim 7 or 8, wherein said inflammatory disease is selected from the group consisting of an inflammatory bowel disease, rheumatoid arthritis, psoriasis, osteoarthritis, diabetes (type I and II), tissue or organ rejection, multiple sclerosis, periodontal inflammation, periodontitis, pigmented villonodular synovitis, hepatitis, sinusitis, colon cancer, colorectal cancer, colitis-associated colon cancer, sporadic colorectal cancer, coronary artery disease, Sjogren's syndrome (SS), obesity, chronic inflammation, pulmonary sarcoidosis, skin lesions, a CNS inflammatory disease and an autoimmune disease.

10. The IL-34 antisense oligonucleotide for use as claimed in claim 9, wherein said inflammatory disease is an inflammatory bowel disease.

11. The IL-34 antisense oligonucleotide for use as claimed in claim 10, wherein the inflammatory bowel disease is selected from the group consisting of Crohn's disease, gastroduodenal Crohn's disease, Crohn's (granulomatous) colitis, ulcerative colitis, collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, and indeterminate colitis.

12. The IL-34 antisense oligonucleotide of any one of claims 1-3 for use in a method of preventing or treating fibrosis.

13. The IL-34 antisense oligonucleotide for use as claimed in claim 12, wherein:
(a) the fibrosis is intestinal fibrosis; or
(b) the fibrosis is pulmonary fibrosis; or
(c) the fibrosis is selected from the group consisting of renal fibrosis, cardiac fibrosis, endomyocardial fibrosis, myelofibrosis, retroperitoneal fibrosis, and nephrogenic systemic fibrosis.

14. The IL-34 antisense oligonucleotide for use as claimed in claim 13 part (a), wherein a pharmaceutically effective amount of a pharmaceutical preparation comprising the IL-34 antisense oligonucleotide of any one of claims 1-3 is administered to a patient, optionally wherein the pharmaceutical preparation is administered orally; and/or wherein the patient is human and/or wherein the patient is also suffering from Crohn's disease, inflammatory bowel disease, or ulcerative colitis.

15. The IL-34 antisense oligonucleotide for use as claimed in claim 13 part (b), wherein a pharmaceutically effective amount of a pharmaceutical preparation comprising the IL-34 antisense oligonucleotide of any one of claims 1-3 is administered to a patient, optionally wherein the pharmaceutical preparation is administered orally; and/or wherein the patient is human.

## Patentansprüche

1. IL-34-Antisense-Oligonukleotid, umfassend die Nukleotidsequenz 5'-AGCTGCTCAGTGTGAA-3' (SEQ ID NO: 5).

2. IL-34-Antisense-Oligonukleotid nach Anspruch 1, wobei mindestens eine Nukleosidbindung der Sequenz ausgewählt ist aus der Gruppe bestehend aus einer Phosphorthioatbindung, einer Phosphordithioatbindung, einer Phosphotriesterbindung, einer Alkylphosphonatbindung, einer Aminoalkylphosphotriesterbindung, einer Alkylenphosphonatbindung, einer Phosphinatbindung, einer Phosphoramidatbindung und einer Aminoalkylphosphoramidatbindung, einer Thiophosphoramidatbindung, einer Thionoalkylphosphonatbindung, einer Thionoalkylphosphotriesterbindung, einer Thiophosphatbindung, einer Selenophosphatbindung und einer Boranophosphatbindung, wobei vorzugsweise mindestens eine Internukleosidbindung der Sequenz eine Phosphorthioatbindung ist; oder wobei vorzugsweise alle Internukleosidbindungen der Sequenz Phosphorthioatbindungen sind.

3. IL-34-Antisense-Oligonukleotid nach einem der Ansprüche 1 oder 2, wobei das Antisense-Oligonukleotid eine Länge von 20 bis 30, 30 bis 40 oder 22 bis 40 Nukleotiden aufweist.

4. Pharmazeutisch akzeptable Zusammensetzung, umfassend ein IL-34-Antisense-Oligonukleotid nach einem der Ansprüche 1 bis 3 und einen pharmazeutisch akzeptablen Träger.

5. Pharmazeutische Zusammensetzung nach Anspruch 4, wobei die pharmazeutische Zusammensetzung zur topischen, parenteralen, oralen, pulmonalen, intratrachealen, intranasalen, transdermalen oder intraduodenalen Verabreichung geeignet ist, wobei die pharmazeutische Zusammensetzung vorzugsweise zur oralen Verabreichung geeignet ist.

6. IL-34-Antisense-Oligonukleotid nach einem der Ansprüche 1 bis 3 zur Verwendung als Arzneimittel.

7. IL-34-Antisense-Oligonukleotid nach einem der Ansprüche 1 bis 3 zur Verwendung bei der Behandlung einer entzündlichen Erkrankung.

8. IL-34-Antisense-Oligonukleotid zur Verwendung nach Anspruch 7, wobei die entzündliche Erkrankung mit veränderter IL-34-Expression assoziiert ist.

9. IL-34-Antisense-Oligonukleotid zur Verwendung nach Anspruch 7 oder 8, wobei die entzündliche Erkrankung ausgewählt ist aus der Gruppe bestehend aus entzündlicher Darmerkrankung, rheumatoider Arthritis, Psoriasis, Osteoarthritis, Diabetes (Typ I und II), Gewebe- oder Organabstoßung, multipler Sklerose, Zahnfleischentzündung, Parodontitis, pigmentierter villonodulärer Synovitis, Hepatitis, Sinusitis, Colonkrebs, Colorektalkrebs, Colitis-assoziiertem Colonkrebs, sporadischem Colorektalkrebs, koronarer Arterienkrankheit oder Sjögren-Syndrom (SS), Fettleibigkeit, chronischer Entzündung, Lungensarkoidose, Hautläsionen, einer entzündlichen Erkrankung des ZNS und einer Autoimmunerkrankung.

10. IL-34-Antisense-Oligonukleotid zur Verwendung nach Anspruch 9, wobei die entzündliche Erkrankung eine entzündliche Darmerkrankung ist.

11. IL-34-Antisense-Oligonukleotid zur Verwendung nach Anspruch 10, wobei die entzündliche Darmerkrankung ausgewählt ist aus der Gruppe bestehend aus Morbus Crohn, gastroduodenalem Morbus Crohn, Crohn's (granulomatöser) Kolitis, Colitis ulcerosa, kollagener Colitis, lymphatischer Colitis, ischämischer Colitis, Diversionscolitis, Morbus Behçet, mikroskopischer Colitis, ulzerativer Proktitis, Proktosigmoiditis, Jejunoileitis, linksseitiger Colitis, Pancolitis, Ileocolitis, Ileitis und unbestimmter Colitis.

12. IL-34-Antisense-Oligonukleotid nach einem der Ansprüche 1 bis 3 zur Verwendung bei einem Verfahren zur Vorbeugung oder Behandlung von Fibrose.

13. IL-34-Antisense-Oligonukleotid zur Verwendung nach Anspruch 12, wobei:
(a) die Fibrose eine Darmfibrose ist; oder
(b) die Fibrose Lungenfibrose ist; oder
(c) die Fibrose ausgewählt ist aus der Gruppe bestehend aus Nierenfibrose, Herzfibrose, Endomyokardfibrose, Myelofibrose, retroperitonealer Fibrose und nephrogener systemischer Fibrose.

14. IL-34-Antisense-Oligonukleotid zur Verwendung nach Anspruch 13, Teil (a), wobei eine pharmazeutisch wirksame Menge eines pharmazeutischen Präparates, umfassend das IL-34-Antisense-Oligonukleotid nach einem der Ansprüche 1 bis 3, einem Patient verabreicht wird, wobei das pharmazeutische Präparat gegebenenfalls oral verabreicht wird; und/oder wobei der Patient ein Mensch ist und/oder wobei der Patient ebenfalls an Morbus Crohn, entzündlicher Darmerkrankung oder Colitis ulcerosa leidet.

15. IL-34-Antisense-Oligonukleotid zur Verwendung nach Anspruch 13, Teil (b), wobei eine pharmazeutisch wirksame Menge eines pharmazeutischen Präparates, umfassend das IL-34-Antisense-Oligonukleotid nach einem der Ansprüche 1 bis 3, einem Patient verabreicht wird, wobei das pharmazeutische Präparat gegebenenfalls oral verabreicht wird; und/oder wobei der Patient ein Mensch ist.

## Revendications

1. Oligonucléotide antisens d'IL-34 comprenant la séquence nucléotidique 5'-AGCTGCTCAGTGTGAA-3' (SEQ ID NO : 5).

2. Oligonucléotide antisens d'IL-34 selon la revendication 1, dans lequel au moins une liaison nucléosidique de la séquence est choisie dans le groupe constitué d'une liaison phosphorothioate, une liaison phosphorodithioate, une liaison phosphotriester, une liaison alkylphosphonate, une liaison aminoalkylphosphotriester, une liaison alkylènephosphonate, une liaison phosphinate, une liaison phosphoramidate et une liaison aminoalkylphosphoramidate, une liaison thiophosphoramidate, une liaison thionoalkylphosphonate, une liaison thionoalkylphosphotriester, une liaison thiophosphate, une liaison sélénophosphate et une liaison boranophosphate, de préférence dans lequel au moins une liaison internucléosidique de la séquence est une liaison phosphorothioate ; ou de préférence dans lequel toutes les liaisons internucléosidiques de la séquence sont des liaisons phosphorothioate.

3. Oligonucléotide antisens d'IL-34 selon l'une quelconque des revendications 1 ou 2 dans lequel l'oligonucléotide antisens est de 20 à 30, 30 à 40, ou 22 à 40 nucléotides de longueur.

4. Composition pharmaceutiquement acceptable comprenant l'oligonucléotide antisens d'IL-34 selon l'une quelconque des revendications 1 à 3 et un véhicule pharmaceutiquement acceptable.

5. Composition pharmaceutique selon la revendication 4, la composition pharmaceutique étant adaptée pour administration topique, parentérale, orale, pulmonaire, intratrachéale, intranasale, transdermique ou intraduodénale, la composition pharmaceutique étant de préférence adaptée pour administration orale.

6. Oligonucléotide antisens d'IL-34 selon l'une quelconque des revendications 1 à 3 pour utilisation en tant que médicament.

7. Oligonucléotide antisens d'IL-34 selon l'une quelconque des revendications 1 à 3 pour utilisation dans le traitement d'une maladie inflammatoire.

8. Oligonucléotide antisens d'IL-34 pour utilisation selon la revendication 7, ladite maladie inflammatoire étant associée à une expression altérée d'IL-34.

9. Oligonucléotide antisens d'IL-34 pour utilisation selon la revendication 7 ou 8, ladite maladie inflammatoire étant choisie dans le groupe constitué de : une maladie inflammatoire chronique de l'intestin, la polyarthrite rhumatoïde, le psoriasis, l'arthrose, le diabète (type I et II), un rejet de tissu ou d'organe, la sclérose en plaques, une inflammation parodontale, une parodontite, la synovite villonodulaire pigmentée, une hépatite, une sinusite, un cancer du côlon, un cancer colorectal, un cancer du côlon associé à une colite, un cancer colorectal sporadique, une maladie coronarienne, le syndrome de Sjögren (SS), l'obésité, une inflammation chronique, la sarcoïdose pulmonaire, des lésions cutanées, une maladie inflammatoire du SNC et une maladie auto-immune.

10. Oligonucléotide antisens d'IL-34 pour utilisation selon la revendication 9, ladite maladie inflammatoire étant une maladie inflammatoire chronique de l'intestin.

11. Oligonucléotide antisens d'IL-34 pour utilisation selon la revendication 10, la maladie inflammatoire chronique de l'intestin étant choisie dans le groupe constitué de la maladie de Crohn, la maladie de Crohn gastroduodénale, la colite de Crohn (granulomateuse), la rectocolite hémorragique, la colite collagénique, la colite lymphocytaire, la colite ischémique, la colite de diversion, la maladie de Behçet, la colite microscopique, la proctite ulcéreuse, la proctosigmoïdite, la jéjunoiléite, la colite côté gauche, la pancolite, l'iléocolite, l'iléite et une colite indéterminée.

12. Oligonucléotide antisens d'IL-34 selon l'une quelconque des revendications 1 à 3 pour utilisation dans un procédé de prévention ou de traitement de la fibrose.

13. Oligonucléotide antisens d'IL-34 pour utilisation selon la revendication 12, où :
(a) la fibrose est la fibrose intestinale ; ou
(b) la fibrose est la fibrose pulmonaire ; ou
(c) la fibrose est choisie dans le groupe constitué de la fibrose rénale, la fibrose cardiaque, la fibrose endomyocardique, la myélofibrose, la fibrose rétropéritonéale et la fibrose systémique néphrogénique.

14. Oligonucléotide antisens d'IL-34 pour utilisation selon la revendication 13 partie (a), une quantité pharmaceutiquement efficace d'une préparation pharmaceutique comprenant l'oligonucléotide antisens d'IL-34 selon l'une quelconque des revendications 1 à 3 étant administrée à un patient, la préparation pharmaceutique étant facultativement administrée par voie orale ; et/ou le patient étant humain et/ou le patient souffrant également de la maladie de Crohn, d'une maladie inflammatoire chronique de l'intestin ou de la recto-colite hémorragique.

15. Oligonucléotide antisens d'IL-34 pour utilisation selon la revendication 13 partie (b), une quantité pharmaceutiquement efficace d'une préparation pharmaceutique comprenant l'oligonucléotide antisens d'IL-34 selon l'une quelconque des revendications 1 à 3 étant administrée à un patient, la préparation pharmaceutique étant facultativement administrée par voie orale ; et/ou le patient étant humain.
